(19) Europäisches Patentamt
European Patent Office
Office européen des brevets

(11) EP 4 286 583 A1

(12) **EUROPEAN PATENT APPLICATION**
published in accordance with Art. 153(4) EPC

(43) Date of publication:
**06.12.2023 Bulletin 2023/49**

(21) Application number: **22752706.6**

(22) Date of filing: **07.02.2022**

(51) International Patent Classification (IPC):
***D21F 7/00*** *(2006.01)* ***C02F 1/00*** *(2023.01)*

(52) Cooperative Patent Classification (CPC):
**C02F 1/00; D21F 7/00**

(86) International application number:
**PCT/JP2022/004611**

(87) International publication number:
**WO 2022/172882 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.02.2021 JP 2021021053**

(71) Applicant: **Kurita Water Industries Ltd.**
**Tokyo 164-0001 (JP)**

(72) Inventors:
- **TOYOOKA, Yasuhiro**
  **Tokyo 164-0001 (JP)**
- **TAGASHIRA, Masaki**
  **Tokyo 164-0001 (JP)**
- **KATSURA, Hiroki**
  **Tokyo 164-0001 (JP)**
- **HARADA, Kaname**
  **Tokyo 164-0001 (JP)**
- **HIDAKA, Katsuhiko**
  **Tokyo 164-0001 (JP)**

(74) Representative: **TBK**
**Bavariaring 4-6**
**80336 München (DE)**

(54) **ESTIMATION DEVICE, ESTIMATION SYSTEM, ESTIMATION PROGRAM, AND ESTIMATION METHOD**

(57) To provide an inference apparatus, an inference system, an inference program, and an inference method which enable an occurrence of trouble in a water system and quality of a product produced via the water system to be quantitatively inferred. According to an aspect of the present invention, an inference apparatus for inferring a potential result which may arise in a water system or which may be derived from the water system and may arise in the future is provided. The inference apparatus includes: a parameter information acquiring unit; a relational model information acquiring unit; and an inferring unit. The parameter information acquiring unit acquires parameter information which includes two or more of one type of parameter among: a water quality parameter related to water quality of the water system; a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system. The relational model information acquiring unit acquires relational model information which is created in advance and which indicates a relationship between the potential result or an index related to the potential result and the two or more parameters. The inferring unit infers the potential result or the index related to the potential result based on the parameter information and the relational model information.

Fig. 1

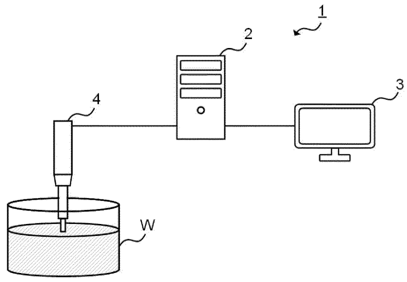


EP 4 286 583 A1

## Description

### Technical Field

The present invention relates to an estimation device (inference apparatus), an estimation system (inference system), an estimation program (inference program), and an estimation method (inference method).

### Background Art

Various products including paper products are produced in processes involving a water system. In such processes, from the perspective of preventing or reducing occurrence of trouble and adverse effects on products during operations, it is standard practice to predict trouble in advance.

For example, in the production of paper products, present values or trends in variation of parameters empirically known to cause trouble or affect quality of the paper products are checked in order to control various operational conditions and chemicals to be added to the water system.

For example, PTL1 proposes a method of measuring two or more water quality parameters related to water quality in a water system for producing paper and performing water treatment of the water system based on measured values of the water quality parameters. In addition, according to the method described in PTL1, high quality paper can be produced.

### Citation List

### Patent Literature

PTL1: International Publication No. WO 2012/070644

### Summary of Invention

### Technical Problem

PTL1 does not disclose quantitatively inferring an occurrence of trouble or quality of a paper product in the water system for producing paper. On the other hand, in order to more accurately control various operational conditions and an amount of chemicals to be added in the system, an occurrence of trouble or quality of a paper product in the water system must be quantitatively inferred.

In consideration of the circumstances described above, an object of the present invention is to provide an inference apparatus, an inference system, an inference program, and an inference method which enable an occurrence of trouble in a water system and quality of a product produced via the water system to be quantitatively inferred.

### Solution to Problem

According to an aspect of the present invention, an inference apparatus for inferring a potential result which may arise in a water system or which may be derived from the water system and may arise in the future is provided. The inference apparatus includes: a parameter information acquiring unit; a relational model information acquiring unit; and an inferring unit. The parameter information acquiring unit acquires parameter information which includes two or more of one type of parameter among: a water quality parameter related to water quality of the water system; a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system. The relational model information acquiring unit acquires relational model information which is created in advance and which indicates a relationship between the potential result or an index related to the potential result and the two or more parameters. The inferring unit infers the potential result or the index related to the potential result based on the parameter information and the relational model information.

The present invention may be provided in each of the aspects described below.

The inference apparatus described above, wherein the relational model is a model obtained from a regression analysis, a time-series analysis, a decision tree, a neural network, Bayes, clustering, or ensemble learning between a prior confirmation result corresponding to the potential result or an index related to the prior confirmation result and the two or more parameters.

The inference apparatus described above, wherein the water system is a water system in a process of producing a paper product.

The inference apparatus described above, wherein the water quality parameter is one or more parameters selected from the group consisting of pH, electrical conductivity, an oxidation-reduction potential, a zeta potential, turbidity, temperature, a foam height, a biochemical oxygen demand (BOD), a chemical oxygen demand (COD), absorbance, color, a particle size distribution, an agglomeration degree, an amount of foreign matter, a foaming area on a water surface, an area of underwater contamination, an amount of bubbles, an amount of glucose, an amount of organic acids, an amount of starch, an amount of calcium, an amount of total chlorine, an amount of free chlorine, an amount of dissolved oxygen, a cationic demand, an amount of hydrogen sulfide, an amount of hydrogen peroxide, and a respiration rate of microorganisms in the water system.

The inference apparatus described above, wherein the control parameter is one or more parameters selected from the group consisting of an operating speed (a papermaking speed) of a paper machine, a rotational speed of a filter cloth of a raw material dehydrator, a rotational speed of a filter cloth of a cleaning machine, an additive amount of chemicals to the water system, an additive amount of chemicals relative to raw materials to be added to the water system, an additive amount of chemicals relative to facilities related to the water system, an amount of vapor for heating, temperature of vapor for heating, pressure of vapor for heating, a flow rate from a headbox, nip pressure of a press part, felt vacuum pressure of a press part, a blending ratio of papermaking raw material, a blended amount of waste sheets of papermaking raw material, a mesh size of a screen for papermaking raw material, a gap distance between a rotor and a stator of a beater, freeness, and a degree of beating.

The inference apparatus described above, wherein the result parameter is one or more parameters selected from the group consisting of a unit weight (grammage) of the paper product, a yield, white water concentration, moisture content of the paper product, an amount of vapor in a facility producing the paper product, temperature of vapor in a facility producing the paper product, pressure of vapor in a facility producing the paper product, thickness of paper product, concentration of ash in the paper product, a type of defect of the paper product, the number of defects in the paper product, a timing of paper breakage in a process, freeness, a degree of beating, and an amount of aeration.

An inference system for inferring a potential result which may arise in a water system or which may be derived from the water system and may arise in the future, the inference system including: a parameter information acquiring unit; a relational model information acquiring unit; and an inferring unit, wherein the parameter information acquiring unit acquires parameter information which includes two or more of one type of parameter among: a water quality parameter related to water quality of the water system; a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system, the relational model information acquiring unit acquires relational model information which is created in advance and which indicates a relationship between the potential result or an index related to the potential result and the two or more parameters, and the inferring unit infers the potential result or the index related to the potential result based on the parameter information and the relational model information.

An inference program for inferring a potential result which may arise in a water system or which may be derived from the water system and may arise in the future, the inference program causing a computer to function as: a parameter information acquiring unit; a relational model information acquiring unit; and an inferring unit, wherein the parameter information acquiring unit acquires parameter information which includes two or more of one type of parameter among: a water quality parameter related to water quality of the water system; a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system, the relational model information acquiring unit acquires relational model information which is created in advance and which indicates a relationship between the potential result or an index related to the potential result and the two or more parameters, and the inferring unit infers the potential result or the index related to the potential result based on the parameter information and the relational model information.

An inference method of inferring a potential result which may arise in a water system or which may be derived from the water system and may arise in the future, the inference method including: a parameter information acquiring step; a relational model information acquiring step; and an inferring step, wherein in the parameter information acquiring step, parameter information which includes two or more of one type of parameter among: a water quality parameter related to water quality of the water system; a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system is acquired, in the relational model information acquiring step, relational model information which is created in advance and which indicates a relationship between the potential result or an index related to the potential result and the two or more parameters is acquired, and in the inferring step, the potential result or the index related to the potential result is inferred based on the parameter information and the relational model information.

It is needless to say that the present invention is not limited to the above.

According to the present invention, an occurrence of trouble in a water system and quality of a product produced via the water system can be quantitatively inferred.

Brief Description of Drawings

[Fig. 1] Fig. 1 is a schematic diagram of an inference system according to a present embodiment.

[Fig. 2] Fig. 2 is a schematic view showing a functional configuration of an inference apparatus according to the present embodiment.

[Fig. 3] Fig. 3 is a schematic diagram showing a hardware configuration of the inference apparatus according to the present embodiment.

[Fig. 4] Fig. 4 shows plots of the number A of occurrences of trouble of a total of 30 data sets against an index a related to a potential result.

[Fig. 5] Fig. 5 is a graph showing a magnitude of influence of each parameter with respect to the index a related to the potential result.

[Fig. 6] Fig. 6 is a flowchart of an inference method according to the present embodiment.

[Fig. 7] Fig. 7 is a schematic view of a facility which produces paper according to Example 1.

[Fig. 8] Fig. 8 shows plots of the number of defects of a total of 572 data sets according to Example 1 against a defect index $\alpha$.

[Fig. 9] Fig. 9 is a graph showing a magnitude of influence of each parameter with respect to the defect index.

[Fig. 10] Fig. 10 shows plots of an original unit of usage of a paper strengthening agent of a total of 60 data sets according to Example 2 against a paper strength index value.

[Fig. 11] Fig. 11 is a graph showing a magnitude of influence of each parameter with respect to the paper strength index.

[Fig. 12] Fig. 12 is a graph showing a change over time of occurrences of paper breakage and a paper breakage index.

[Fig. 13] Fig. 13 is a graph showing a change over time of the occurrences of paper breakage and the paper breakage index.

[Fig. 14] Fig. 14 is a schematic view of a facility which produces paper according to Example 4.

[Fig. 15] Fig. 15 shows plots of the number of defects of a total of 647 data sets for relational model creation according to Example 4 against values of a defect index $\beta$.

[Fig. 16] Fig. 16 shows plots of the number of defects of a total of 255 data sets for accuracy validation according to Example 4 against the values of the defect index $\beta$.

[Fig. 17] Fig. 17 shows plots indicating a change over time of a defect index calculated from the total of 255 data sets for the accuracy validation according to Example 4.

[Fig. 18] Fig. 18 is a graph showing a magnitude of influence of each parameter with respect to the defect index $\beta$.

[Fig. 19] Fig. 19 shows plots of the number of defects of a total of 631 data sets for relational model creation according to Example 5 against values of a defect index $\gamma$.

[Fig. 20] Fig. 20 shows plots of the number of defects of a total of 255 data sets for accuracy validation according to Example 5 against the values of the defect index $\gamma$.

[Fig. 21] Fig. 21 shows plots indicating a change over time of a defect index calculated from the total of 271 data sets for the accuracy validation according to Example 5.

[Fig. 22] Fig. 22 is a graph showing a magnitude of influence of each parameter with respect to the defect index $\gamma$.

[Fig. 23] Fig. 23 shows plots of the number of defects of a total of 1216 data sets for relational model creation according to Example 6 against values of a defect index $\delta$.

[Fig. 24] Fig. 24 shows plots of the number of defects of a total of 490 data sets for accuracy validation according to Example 6 against the values of the defect index $\delta$.

[Fig. 25] Fig. 25 is a graph showing a magnitude of influence of each parameter with respect to the defect index $\delta$.

[Fig. 26] Fig. 26 shows plots of the number of defects of a total of 1503 data sets for relational model creation according to Example 7 against a defect index $\varepsilon$.

[Fig. 27] Fig. 27 shows plots of the number of defects of a total of 537 data sets for accuracy validation according to Example 7 against the defect index ε.

[Fig. 28] Fig. 28 is a graph showing a magnitude of influence of each parameter with respect to the defect index ε.

Description of Embodiment

Hereinafter, an embodiment of the present invention will be described with reference to the drawings. Various constituent elements shown in the embodiment described below can be combined with each other.

A program for realizing software according to the present embodiment may be provided as a non-transitory computer-readable medium, provided so as to be downloadable from an external server, or provided so as to be run on an external computer such that functions thereof are realized on a client terminal (so-called cloud computing).

In addition, a "unit" as referred to in the present embodiment may include, for example, a combination of hardware resources implemented by a circuit in a broad sense and information processing by software which may be specifically realized by such hardware resources. Furthermore, while various kinds of information are handled in the present embodiment, for example, such information are to be represented by a physical value of a signal value representing a voltage or a current, a level of a signal value as an aggregate of binary bits constituted of 0 or 1, or a quantum superposition (a so-called quantum bit), and communication and calculations can be executed on the circuit in a broad sense.

Moreover, the circuit in a broad sense is a circuit which is realized by at least appropriately combining a circuit, circuitry, a processor, a memory, and the like. In other words, the circuit in a broad sense includes an application specific integrated circuit (ASIC), a programmable logic device (for example, a simple programmable logic device (SPLD), a complex programmable logic device (CPLD), or a field programmable gate array (FPGA)), and the like.

<Inference system>

An inference system according to the present embodiment is an inference system for inferring a potential result which may arise in a water system or which may be derived from the water system and may arise in the future. Specifically, the estimation system includes: a parameter information acquiring unit; a relational model information acquiring unit; and an inferring unit. Among these units, the parameter information acquiring unit acquires parameter information which includes two or more of one type of parameter among: a water quality parameter related to water quality of the water system; a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system. The relational model information acquiring unit acquires relational model information which is created in advance and which indicates a relationship between the potential result or an index related to the potential result and the two or more parameters. The inferring unit infers the potential result or the index related to the potential result based on the parameter information and the relational model information.

In addition, although not essential constituent elements, the inference system according to the present embodiment may include one of or both a relational model creating unit and an output unit. Note that an inference system including all of these units will be mainly explained with reference to Fig. 1 to be described below.

[Functional configuration of inference system]

Fig. 1 is a schematic diagram of an inference system according to the present embodiment. The inference system 1 includes an inference apparatus 2 and an output apparatus 3.

Among these components, the inference apparatus 2 controls information processing for inference in the inference system 1. Fig. 2 is a schematic view showing a functional configuration of the inference apparatus according to the present embodiment. As shown in Fig. 2, the inference apparatus 2 according to the present embodiment is an inference apparatus for inferring a potential result which may arise in a water system W or which may be derived from the water system W and may arise in the future and includes a parameter information acquiring unit 21, a relational model information acquiring unit 22, and an inferring unit 23. In addition, the inference apparatus 2 according to the present embodiment further includes a second inferring unit 24, a relational model creating unit 25, a second relational model creating unit 26, and a second relational model information acquiring unit 27. While the respective units are described herein as being included inside a single apparatus, alternatively, each unit may be included as a separate apparatus. In addition, while the output apparatus 3 is an example of the output unit and a parameter information measurement apparatus 4 is an example of the parameter information measuring unit, a description will be hereinafter given without particularly distinguishing the apparatuses from the units.

In this case, "a potential result which may arise in the future in a water system" among "a potential result which may arise in a water system or which may be derived from the water system and may arise in the future" refers to a result which arises in the water system when managing or operating the water system and, although results may differ depending on an object water system, examples of results include clogging of the water system, an unintended increase in microorganisms, foam formation, generation or worsening of odor, an increase in biochemical oxygen demand (BOD), an increase in (COD), an increase in suspended solids, a rise in turbidity, a rise in chromaticity, a drop in transparency, an increase in an amount of dewatered sludge, an increase in moisture content of sludge cake, a decline in efficiency of heat exchanger, a decline in efficiency of freezer, a decline in efficiency of chiller, a decline in efficiency of cooling tower, an increase in backwash frequency of filter media of filter or activated carbon, an increase in replacement frequency of filter media of filter or activated carbon, an increase in cleaning frequency of membranes (MF membrane, UF membrane, RO membrane, and the like), an increase in replacement frequency of membranes, an increase in regeneration frequency of ion exchange resins, an increase in replacement frequency of ion exchange resins, corrosion of facilities and piping (due to microorganism contamination or residual chlorine, and an increase in chemicals (an antifoam agent, a draining agent or a flocculant, a coagulant, a COD reducing agent, a chelating agent, a biological treatment nutrient, a dehydrating agent for sludge, membrane contamination inhibitor, slime control agent, scale retarder, a corrosion-preventing agent, a cleaning agent, a pH adjuster (acid, alkali), a buffering agent, an oxidizing agent, a reductant, and an ion-exchange resin regeneration chemical agent). In addition, "a potential result which may be derived from the water system and may arise in the future" refers to a result which arises outside of the water system in relation to the water system when managing or operating the water system and, although results may differ depending on an object water system, examples of results include a decline in performance of a product, a decline in yield of the product, an increase in unwanted by-products or the like, and a change in odor of the product. Furthermore, "an index related to a potential result" may be an index (for example, a function with two or more parameters) having a certain correlative relationship with the potential result and may be an index uniquely created by a user (an operator or the like) of the inference system instead of a generally known index.

In addition, examples of "a potential result which may arise in the future in a water system" in a process of producing paper include contamination of a paper machine, contamination of a papermaking approach system, contamination of a white water recovery system, air entrainment of pump, screen blocking, a decline in papermaking speed, poor freeness in a wire part, incomplete dehydration in a press part, poor drying in a dryer part, odor in the water system, poor separation in a dryer process, contamination of a broke system, and contamination of a raw material system. Examples of "a potential result which may be derived from the water system and may arise in the future" in a process of producing paper includes, for example, results related to a paper product produced from the water system (the number of defects, paper strength, a joint rate, a sizing degree, air permeability, smoothness, an ash content, a tone, whiteness, formation, odor, causticizing efficiency, calcination rate, a kappa value, freeness, and a moisture percentage) and phenomena which may occur outside of the water system (for example, paper breakage in the press part to the dryer part).

[Functions of inference system]

Hereinafter, a function of each unit of the inference system 1 will be described in specific terms.

[Parameter information acquiring unit]

The parameter information acquiring unit 21 acquires parameter information which includes two or more of one type of parameter among: a water quality parameter related to water quality of a water system W; a control parameter related to the water system W, a facility related to the water system W, or a control condition of a raw material to be added to the water system W; and a result parameter which is a parameter having a different meaning from the potential result and which is related to a result having arisen in the water system W, a facility related to the water system W, or a raw material to be added to the water system W or a result having been derived and arisen from the water system W, a facility related to the water system W, or a raw material to be added to the water system W.

Note that the water system in this case is not limited to a water system present in a single tank or a single flow path or a water system in which a continuous flow is present, and water systems with a plurality of tanks or flow paths such as a water system in which a branch is present in a flow path, a water system with a confluence of a plurality of flow paths, a water system which involves a movement in batch units from one tank to another, and a water system in which mid-flow processing is performed are also considered single water systems. In addition, when a water system related to water quality parameters, control parameters, or result parameters are divided according to tanks or the like, water quality parameters, control parameters, or result parameters with respect to a part of the water system may be used or water quality parameters, control parameters, or result parameters with respect to the entire water system may be used.

Water quality parameters are not particularly limited as long as the parameters are related to a water quality of the water system W. In addition, the control parameters are not particularly limited as long as the parameters are related to the water system W, a facility related to the water system W, or a control condition of a raw material to be added to the water system W. Furthermore, the control parameters are not particularly limited as long as the parameters have a different meaning from the potential result and which is related to a result having arisen in the water system W, a facility related to the water system W, or a raw material to be added to the water system W or a result having been derived and arisen from the water system W, a facility related to the water system W, or a raw material to be added to the water system W. Note that while "have a different meaning from the potential result" includes cases where assessment indices (for example, physical amount) differs (for example, when one of the assessment indices is a length and the other assessment index is a mass) and cases where the assessment index is the same but objects of assessment differ from each other (for example, a mass of paper and a mass of an additive) or locations of measurement differ from each other (for example, an oxidation-reduction potential of a papermaking raw material system and an oxidation-reduction potential of a papermaking system), cases where meanings of results differ from each other (for example, "have a different meaning from the potential result" does not include a case where an oxidation-reduction potential is positive and a case where an oxidation-reduction potential is negative).

Hereinafter, specific examples of the water quality parameter, the control parameter, and the result parameter in a case where the water system W is a water system in a process of producing a paper product will be described.

As the water quality parameter, for example, one or more water quality parameters selected from the group consisting of pH, electrical conductivity, an oxidation-reduction potential, a zeta potential, turbidity, temperature, a foam height, a biochemical oxygen demand (BOD), a chemical oxygen demand (COD), absorbance (for example UV absorbance), color (for example, an RGB value), a particle size distribution, an agglomeration degree, an amount of foreign matter, a foaming area on a water surface, an area of underwater contamination, an amount of bubbles, an amount of glucose, an amount of organic acids, an amount of starch, an amount of calcium, an amount of total chlorine, an amount of free chlorine, an amount of dissolved oxygen, a cationic demand, an amount of hydrogen sulfide, an amount of hydrogen peroxide, and a respiration rate of microorganisms in the water system is preferably used.

As the control parameter, for example, one or more control parameters selected from the group consisting of an operating speed (a papermaking speed) of a paper machine, a rotational speed of a filter cloth of a raw material dehydrator, a rotational speed of a filter cloth of a cleaning machine, an additive amount of chemicals to the water system, an additive amount of chemicals relative to raw materials to be added to the water system, an additive amount of chemicals relative to facilities related to the water system, an amount of vapor for heating, temperature of vapor for heating, pressure of vapor for heating, a flow rate from a headbox, nip pressure of a press part, felt vacuum pressure of a press part, a blending ratio of papermaking raw material, a blended amount of waste sheets of papermaking raw material, a mesh size of a screen for papermaking raw material, a gap distance between a rotor and a stator of a beater, freeness, and a degree of beating is preferably used. Note that when the water system W is a water system in a process of producing a paper product, examples of "facilities related to the water system" include facilities of wire or felt in the paper machine in which chemicals are directly added.

As the result parameter, for example, one or more result parameters selected from the group consisting of a unit weight (grammage) of a paper product, a yield, white water concentration, moisture content of the paper product, an amount of vapor in a facility producing the paper product, temperature of vapor in a facility producing the paper product, pressure of vapor in a facility producing the paper product, thickness of the paper product, concentration of ash in the paper product, a type of defect of the paper product, the number of defects in the paper product, a timing of paper breakage in a process, freeness, a degree of beating, and an amount of aeration is preferably used. Among these result parameters, as the amount of vapor in a facility producing the paper product, an amount of vapor in a dryer of the paper machine, an amount of vapor in an evaporator of kraft pulp black liquor, an amount of vapor in a black liquor heater of a kraft pulp digester, and an amount of vapor which is injected in order to heat pulp raw material or white water can be used.

Note that there are parameters which originally represent a same matter but are classified according to their purpose into two or more of water quality parameters, control parameters, and result parameter. For example, in a black liquor evaporator, black liquor is heated by indirect heat exchange with vapor generated by a boiler and, as a result of the heating, process vapor is generated from the black liquor. The process vapor is used to heat (thicken) thickened black liquor in a next step. The amount of generated process vapor is a result parameter from the perspective of being generated from black liquor and is also used as a control parameter from the perspective of being used in heating (thickening) thickened black liquor in a next step. In addition, the vapor generated by the boiler in order to heat the black liquor is used as a control parameter. Note that the two or more parameters acquired by the parameter information acquiring unit are acquired so that all of the parameters are not substantially the same. For example, in a case where all of the process vapor generated from the black liquor described above is used to heat (thicken) the thickened black liquor in a next step, the process vapor generated from the black liquor as a result parameter and the amount of vapor to be used to heat (thicken) the thickened black liquor as a control parameter are excluded as the two parameters (parameters other than these two are not used). This is because, in such a case, the process vapor generated from the black liquor as a result parameter and the amount of vapor to be used to heat (thicken) the thickened black liquor as a control parameter are substantially the same. However, in a case where a part of the process vapor generated from the black liquor is used to heat (thicken) the thickened black liquor in a next step, the process vapor generated from the black liquor as a result parameter and the amount of vapor to be used to heat (thicken) the thickened black liquor as a control parameter can be used as the two parameters. This is because, in such a case, the process vapor generated from the black liquor as a result parameter and the amount of vapor to be used to heat (thicken) the thickened black liquor as a control parameter are not substantially the same. Furthermore, in a case where all of the process vapor generated from the black liquor described above is used to heat (thicken) the thickened black liquor in a next step, in addition to using the process vapor generated from the black liquor as a result parameter and the amount of vapor to be used to heat (thicken) the thickened black liquor as a control parameter as the two parameters, further combining another parameter such as the pH of the water system or the like as a water quality parameter enables the plurality of parameters to be substantially the same. In addition, for example, while freeness and degree of beating are the same parameters, freeness and degree of beating can be included in both control parameters and result parameters.

The parameters may be quantitative or qualitative. When using a qualitative parameter, a numerical value may be assigned to the parameter and the parameter may be handled as quantitative data.

Note that the water quality parameter, the control parameter, and the result parameter are respectively concepts encompassing a plurality of parameters. With respect to "parameter information which includes two or more parameters", the two or more parameters included in the parameter information are respectively independent and can be selected from the respective parameters of the water quality parameter, the control parameter, and the result parameter, and two or more parameters (for example, pH and temperature of water) may be selected from only one of the water quality parameter, the control parameter, and the result parameter or two or more parameters may be selected from a combination of two or three (for example, pH, press pressure of a press part, and thickness of paper product) of the water quality parameter, the control parameter, and the result parameter. However, exact same parameters (for example, pH of water at location A and pH of water at location A) are not to be selected (however, for example, pH of water at location A and pH of water at location B which represent different measurement locations may be selected).

[Relational model information acquiring unit]

The relational model information acquiring unit 22 acquires relational model information which is created in advance and which indicates a relationship between the potential result or an index related to the potential result and the two or more parameters.

The relational model is created in advance and indicates a relationship between the potential result or an index related to the potential result and the two or more parameters. Note that "in advance" means prior to estimating the potential result or an index related to the potential result and may be any time prior to estimating the potential result or an index related to the potential result including during an operation in the water system W or prior to actual operation.

Examples of the relational model include, but are not particularly limited to, a function or a look-up table indicating a relationship between the potential result or an index related to the potential result and two or more parameters and a trained model representing a relationship between the potential result or an index related to the potential result and two or more parameters.

It is assumed that the two or more parameters included in the parameter information acquired by the parameter information acquiring unit 21 and the parameters included in the parameter information used in the relational model have two or more parameters that are common to each other. As described earlier, the water quality parameter, the control parameter, and the result parameter are respectively concepts encompassing a plurality of parameters. "Have two or more parameters that are common to each other" may mean two or more of any parameters (for example, two or more of only water quality parameters; pH and temperature of water) among the water quality parameter, the control parameter, and the result parameter being common, a combination of the water quality parameter, the control parameter, and the result parameter (for example, one water quality parameter and one control parameter; for example, pH of water and press pressure of the press part) being common, or all of the water quality parameter, the control parameter, and the result parameter (for example, one water quality parameter, one control parameter, and one result parameter) being common.

[Inferring unit]

The inferring unit 23 infers the potential result or the index related to the potential result based on the parameter information and the relational model information.

Specifically, in the inferring unit 23, the potential result or the index related to the potential result is inferred (calculated) by inputting present parameter information of the water system W to the relational model created in advance and performing substitution, collation, or the like to the relational model.

[Second inferring unit]

The second inferring unit 24 infers, when an index related to the potential result and not the potential result itself is inferred by the inferring unit 23, the potential result from the index. Note that when the second inferring unit 24 is provided, the inferring unit 23 will be referred to as a "first inferring unit" for the sake of convenience.

When an index related to the potential result (hereinafter, also referred to as a "related index") is to be inferred by the first inferring unit 23, the potential result must be inferred from the related index. Specifically, the related index is input to a second relational model prepared in advance to infer the potential result. Note that when the second relational model is used, the relational model used by the first inferring unit 23 will be referred to as a "first relational model" for the sake of convenience.

In the case of inferring the potential result, for example, the related index may be provided with a threshold and it can be inferred that trouble is to occur when the related index is larger (or smaller) than the threshold.

In the case of inferring the possibility of an occurrence of trouble, for example, a plurality of thresholds may be set to the related index in order to provide a division into three stages, and it can be inferred that: trouble will surely occur when the related index is in a first stage; trouble may possibly occur when the related index is in a second stage; and trouble will surely not occur when the related index is in a third stage. Alternatively, a relationship between the related index and an occurrence probability of trouble can be converted into a function or a learned model from statistical data of actual operation to calculate a probability of an occurrence of trouble.

[Relational model creating unit]

The relational model creating unit 25 creates a relational model. The relational model may be acquired by the relational model information acquiring unit 22 and used by the inferring unit 23 to infer the potential result or an index related to the potential result.

For example, a relational model is created as follows. Prior to inferring the potential result or the related index, a premeasured result corresponding to the potential result or a premeasured index related to a prior result is measured. In addition, two or more of any one parameter among the water quality parameter, the control parameter, and the result parameter are measured in a same water system. A data set of the premeasured result or the premeasured index and the parameters are prepared in plurality so that variations arise in the premeasured result or the premeasured index and the parameters by, for example, changing a date or time at which measurement is performed. Next, assuming that the premeasured result or the premeasured index is a function of the two or more parameters, the function is compared with the premeasured result or the premeasured index to determine a form and coefficients of the function and a relational model is constructed. In this case, when determining coefficients of the function by a comparison between the function of the two or more parameters and the premeasured result or the premeasured index, a regression analysis method (a linear model, a generalized linear model, a generalized linear mixed model, ridge regression, lasso regression, an elastic net, support vector regression, projection pursuit regression, and the like), a time-series analysis (a VAR model, a SVAR model, an ARIMAX model, a SARIMAX model, a state space model, and the like), a decision tree (a decision tree, a regression tree, a random forest, XGBoost, and the like), a neural network (a simple perceptron, a multilayer perceptron, a DNN, a CNN, an RNN, an LSTM, and the like), Bayes (naive Bayes and the like), clustering (k-means, k-means ++, and the like), and ensemble learning (Boosting, Adaboost, and the like) can be used.

In an embodiment, the relational model is preferably a model obtained from a regression analysis between a prior confirmation result corresponding to the potential result or an index related to the prior confirmation result and the two or more parameters. Note that the number of sample sets when performing a regression analysis is not particularly limited.

The creation of the relational model is preferably performed in a same water system as the water system of which the potential result is to be inferred. In addition, for example, in a case where the water quality of a water system changes significantly even in a same apparatus (for example, a case where pulp that is a papermaking raw material is changed or the like in a papermaking system at a paper mill), a relational model with respect to the water system after the water quality changes is preferably created and used.

From such a viewpoint, every time the potential result or the related index and the two or more parameters are measured on a regular or irregular basis during operation of the water system W, a relational model may be created or a relational model may be updated by adding data.

Note that since the relational model creating unit 25 is not an essential constituent element, the creation of a relational model may be manually performed by, for example, an operator or the like.

[Second relational model creating unit]

The second relational model creating unit 26 creates a second relational model. The second relational model may be acquired by the second relational model information acquiring unit 26 to be described later and used by the second inferring unit 24 to infer the potential result.

In this case, the second relational model refers to a model indicating a relationship between the related index and the potential result. Note that when the second relational model creating unit 26 is provided, the relational model creating unit 25 will be referred to as a "first relational model creating unit" for the sake of convenience.

Examples of the second relational model include, but are not particularly limited to, a function or a look-up table indicating a relationship between the related index and the potential result and a trained model representing a relationship between the related index and the potential result.

For example, a second relational model is created as follows. Prior to inferring the potential result, a premeasured result corresponding to the potential result and a premeasured index related to a prior result are measured. A data set of the premeasured result and the premeasured index are prepared in plurality so that variations arise in the premeasured result and the premeasured index by, for example, changing a date or time at which measurement is performed. Next, an inference model is constructed with the premeasured result as a function of the premeasured index. Alternatively, for example, the second relational model may be constructed by after preparing a plurality of the data set of the premeasured result and the premeasured index, setting a threshold with respect to the related index at a point (premeasured index) where the premeasured result changes significantly.

The creation of the second relational model is preferably performed in a same water system as the water system the potential result of which is to be inferred. In addition, for example, in a case where the water quality of a water system changes significantly even in a same apparatus (for example, a case where pulp that is a papermaking raw material is changed or the like in a papermaking system at a paper mill), a second relational model with respect to the water system after the water quality changes is preferably created and used.

Note that since the second relational model creating unit 26 is not an essential constituent element, the creation of a second relational model may be manually performed by, for example, an operator or the like.

[Second relational model information acquiring unit]

The second relational model information acquiring unit 27 acquires the second relational model. The second relational model may be created by the second relational model creating unit 26.

Note that when the second relational model acquiring unit 27 is provided, the relational model acquiring unit 22 will be referred to as a "first relational model acquiring unit" for the sake of convenience.

[Relational model assessing unit]

The inference system 1 and the inference apparatus 2 may include a relational model assessing unit (not illustrated).

The relational model assessing unit assesses the relational model created by the relational model creating unit 25 and assesses a magnitude of influence of each piece of parameter information with respect to the potential result or the related index.

The magnitude of influence with respect to the relational model differs from one parameter to the next, and even when including a large number of parameters the influence of which with respect to the relational model is small, not only does accuracy not improve but efficiency of calculation or the like may also decline. In consideration thereof, in order to exclude pieces of parameter information with a small influence with respect to the relational model in a relational model information adjusting unit to be described later, the relational model assessing unit assesses a magnitude of influence of each piece of parameter information.

While a method of assessing the magnitude of influence of each piece of parameter information is not particularly limited, for example, when the relational model is represented with the potential result as a linear function of each parameter, a comparative assessment based on a magnitude of an absolute value of a coefficient of the linear function may be performed. For example, pieces of parameter information may be arranged in an order of magnitude of influence with respect to the relational model and pieces of parameter information other than a predetermined number of pieces of parameter information in a descending order of magnitude of influence may be excluded, a predetermined number of pieces of parameter information in an ascending order of magnitude of influence may be excluded, or a threshold may be provided and a relational model adjusting unit may exclude parameters below the threshold, or the like.

[Relational model adjusting unit]

The inference system 1 and the inference apparatus 2 may include a relational model information adjusting unit (not illustrated).

As described above, after the relational model information adjusting unit adjusts parameter information by excluding pieces of parameter information an influence of which with respect to the relational model is small, the relational model information adjusting unit once again instructs the relational model information creating unit 25 to create relational model information.

When providing the relational model assessing unit and the relational model information adjusting unit described above, an assessment by the relational model assessing unit and an adjustment by the relational model information adjusting unit may be performed only once or repetitively performed two or more times.

[Output unit]

The output unit 3 is configured to output at least one of the potential result or the related index calculated by the inferring unit 23 and the potential result inferred by the second inferring unit 24.

For example, the output unit 3 may display the potential result or the related index in a chronological manner (such as a graph of the potential result or the related index against time).

For example, the output unit 3 may output a warning when the potential result or the related index exceeds a certain threshold.

[Parameter information measuring unit]

A parameter information measuring unit 4 measures water quality parameters, control parameters, or result parameters.

While only one parameter information measurement apparatus is described as the parameter information measurement apparatus 4 in Fig. 1 for the sake of convenience, the number of parameter information measurement apparatuses is not limited to this example and two or more parameter information measurement apparatuses may be used.

Measurement apparatuses differ depending on contents of parameters to be measured and various sensors or the like can be selected. As the measurement apparatus, for example, a pH meter, an electrical conductivity meter, an oxidation-reduction potentiometer, a turbidimeter, a thermometer, a level meter for measuring foam height, a COD meter, a UV meter, a particle size distribution analyzer, a flocculation sensor, a digital camera (or a digital video camera), an internal bubble sensor, an absorption photometer, a freeness meter, a dissolved oxygen meter, a zeta potentiometer, a residual chlorine meter, a hydrogen sulfide meter, a retention/freeness meter, a color sensor, and a hydrogen peroxide meter can be used.

Note that in some cases, control parameters and the like directly input in order to control the apparatus are used as it is, and such data can be received via communication from the apparatus or an operator of the apparatus or the like may record the data outside of the apparatus for the purpose of recording the control parameters.

The water system to be an object of the inference system and the like according to the present embodiment is not particularly limited and may be, for example, a water system in a process of producing a paper product. Specifically, a process of producing a paper product includes a cooking process, a washing process, a black liquor-concentration process, and a caustification process. In addition, as water systems other than a water system of a process of producing a paper product, examples of water systems to be an object include various piping, heat exchangers, storage tanks, kilns, and cleaning equipment.

[Hardware configuration of inference system]

Fig. 3 is a schematic diagram showing a hardware configuration of the inference apparatus according to the present embodiment. As shown in Fig. 3, the inference apparatus 2 includes a communicating unit 51, a storage unit 52, and a control unit 53, and these constituent elements are electrically connected via a communication bus 54 inside the inference apparatus 2. Hereinafter, the constituent elements will be further described.

While the communicating unit 51 is preferably wired communication means such as USB, IEEE 1394, Thunderbolt, and wired LAN network communication, the communicating unit 51 can include wireless LAN network communication, mobile communication such as 3G/LTE/5G, Bluetooth (registered trademark) communication, and the like when necessary. In other words, more preferably, the communicating unit 51 is implemented as an assembly of the plurality of communication means described above. Accordingly, exchange of information and commands is executed between the inference apparatus 2 and other devices capable of communicating with the inference apparatus 2.

The storage unit 52 stores various information defined according to the description provided above. The storage unit 52 can be implemented as, for example, a storage device such as a solid state drive (SSD) or a memory such as a random access memory (RAM) which stores information (arguments, arrays, and the like) temporarily necessary in relation to calculations of a program. In addition, the storage unit 52 may be a combination thereof. Furthermore, the storage unit 52 stores various programs which can be read by the control unit 53 to be described later.

The control unit 53 performs processing and control of overall operations related to the inference apparatus 2.

The control unit 53 is, for example, a central processing unit (CPU, not illustrated). The control unit 53 realizes various functions related to the inference apparatus 2 by reading a predetermined program stored in the storage unit 52. In other words, by causing information processing by software (stored in the storage unit 52) to be concretely realized by hardware (the control unit 53), the information processing can be executed as respective functional units in the control unit 53 as shown in Fig. 3. While a single control unit 53 is notated in Fig. 3, actual configurations are not limited thereto and a configuration may be adopted in which a plurality of control units 53 are provided for each function or a single control unit and a plurality of control units may be combined with each other.

[Example of creating a relational model]

Hereinafter, an example of creating a relational model will be described. Specifically, a case where an index a related to a potential result is calculated using a water quality parameter x, a water quality parameter y, and a control parameter z and the index a and the number A of occurrences of trouble (times/day) as the potential result are calculated will be described.

When creating a relational model, the water quality parameter x, the water quality parameter y, and the control parameter z shown in Table 1 below are measured and, at the same time, the number A of occurrences of trouble is also measured to acquire a total of 30 sets of data. The acquired data is shown in Table 1 below.

The index a related to the potential result is represented by the following equation (1), where "parameters" stand for x, y, z, $b_n$ is a coefficient of x, y, z, and $a_0$ and $b_0$ are constants.

$$a = a_0 \sum (b_n \times \text{parameters}) + b_0 \quad \cdots (1)$$

A negative binary regression analysis is performed based on actual measured values of the number A of occurrences of trouble and the index a related to the potential result of equation (1). The index a related to the potential result calculated by the negative binary regression analysis is also shown in Table 1. In addition, Fig. 4 shows plots of the number A of occurrences of trouble of the total of 30 data sets against the index a related to the potential result. A correlation coefficient between the number A of occurrences of trouble and the index a related to the potential result is $r = 0.78$ ($p < 0.05$) and a strong correlation is observed.

[Table 1]

| Data No. | Actual measured value of number A of occurrences (times/day) | Water quality parameter x | Water quality parameter y | Control parameter z | Index a related to potential result |
|---|---|---|---|---|---|
| 1 | 11 | 0 | 430 | 345 | 0.86 |
| 2 | 10 | -3 | 330 | 335 | 1.09 |
| 3 | 14 | -14 | 371 | 246 | 0.69 |
| 4 | 22 | -5 | 315 | 251 | 0.76 |
| 5 | 61 | -40 | 390 | 393 | 1.68 |
| 6 | 12 | 12 | 380 | 387 | 1.09 |
| 7 | 19 | -28 | 381 | 313 | 1.05 |
| 8 | 20 | 21 | 376 | 279 | 0.60 |
| 9 | 32 | -22 | 375 | 378 | 1.40 |
| 10 | 50 | -33 | 380 | 397 | 1.66 |
| 11 | 59 | -44 | 390 | 386 | 1.68 |
| 12 | 8 | 21 | 416 | 367 | 0.83 |
| 13 | 19 | 92 | 421 | 376 | 0.47 |
| 14 | 10 | 43 | 410 | 218 | 0.34 |
| 15 | 19 | -12 | 391 | 215 | 0.55 |

(continued)

| Data No. | Actual measured value of number A of occurrences (times/day) | Water quality parameter x | Water quality parameter y | Control parameter z | Index a related to potential result |
|---|---|---|---|---|---|
| 16 | 13 | 22 | 375 | 372 | 0.94 |
| 17 | 71 | -42 | 381 | 393 | 1.75 |
| 18 | 12 | 33 | 325 | 374 | 0.98 |
| 19 | 9 | 0 | 401 | 223 | 0.51 |
| 20 | 9 | -11 | 410 | 210 | 0.51 |
| 21 | 31 | -5 | 256 | 399 | 1.85 |
| 22 | 45 | -11 | 390 | 381 | 1.24 |
| 23 | 22 | -20 | 395 | 375 | 1.28 |
| 24 | 10 | -1 | 405 | 381 | 1.10 |
| 25 | 10 | -13 | 401 | 268 | 0.70 |
| 26 | 29 | -11 | 401 | 379 | 1.20 |
| 27 | 7 | 111 | 470 | 351 | 0.31 |
| 28 | 10 | -42 | 485 | 318 | 0.92 |
| 29 | 10 | -22 | 455 | 303 | 0.78 |
| 30 | 13 | 29 | 461 | 271 | 0.43 |

In addition, Fig. 5 is a graph showing a magnitude of influence of each parameter with respect to the index a related to the potential result. In this example, it is shown that the magnitudes of influence with respect to the index a related to the potential result are in a descending order of the control parameter z, the water quality parameter x, and the water quality parameter y. By performing a negative binary regression analysis using a standardized score of each parameter, the result shown in Fig. 5 is obtained. Note that a standardized score can be obtained by (individual numerical value - average value)/standard deviation.

Note that a function of the index a related to the potential result used in the regression analysis and the water quality parameter x, the water quality parameter y, and the control parameter z is not limited to the equation (1) described above and a general formula (2) can be used.

$$f(a) = \sum (b_n \times \text{parameters}) + b_0 \quad \cdots (2)$$

where f(a) is any of a, log a, $log\frac{a}{1-a}, \frac{1}{a}, \frac{1}{a^2}$ .

According to the inference system 1 and the inference apparatus 2 described above, an occurrence of trouble in a water system W and quality of a product produced via the water system W can be quantitatively inferred. In particular, even when the number of parameters that influence occurrence of trouble or quality of products is large, each influence can be more accurately taken into consideration and the occurrence of trouble or the quality of products can be predicted.

<Inference program>

An inference program according to the present embodiment is an inference program for inferring a potential result which may arise in a water system or which may be derived from the water system and may arise in the future. Specifically, the inference program causes a computer to function as: a parameter information acquiring unit; a relational model information acquiring unit; and an inferring unit. The parameter information acquiring unit acquires parameter information which includes two or more of one type of parameter among: a water quality parameter related to water quality of a water system, a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system. The relational model information acquiring unit acquires relational model information which is created in advance and which indicates a relationship between the potential result or an index related to the potential result and the two or more parameters. The inferring unit infers the potential result or the index related to the potential result based on the parameter information and the relational model information.

Note that as the parameter information acquiring unit, the relational model information acquiring unit, and the inferring unit, since units similar to those of the inference system described above can be used, a description thereof will be omitted.

<Inference method>

An inference method according to the present embodiment is an inference method for inferring a potential result which may arise in a water system or which may be derived from the water system and may arise in the future. Specifically, the inference method includes: a parameter information acquiring step; a relational model information acquiring step; and an inferring step. In the parameter information acquiring step, parameter information which includes two or more of one type of parameter among: a water quality parameter related to water quality of a water system, a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system is acquired. In the relational model information acquiring step, relational model information which is created in advance and which indicates a relationship between the potential result or an index related to the potential result and the two or more parameters is acquired. In the inferring step, the potential result or the index related to the potential result is inferred based on the parameter information and the relational model information.

Fig. 6 is a flowchart of an inference method according to the present embodiment. As shown in Fig. 6, in a support method according to the present embodiment, parameter information is acquired (parameter information acquiring step S1), relational model information is acquired (relational model information acquiring step S2), and with the parameter information and the relational model information as input information, a potential result or an index related to the potential result is inferred (inferring step S3).

Examples

While the present invention will be described below in more concrete terms by showing examples, it is to be understood that the following examples are not intended to limit the present invention in any way whatsoever.

Example 1

In a water system of a paper producing facility (a continuous water system made up of a raw material system, a papermaking system, and a recovery system), an oxidation-reduction potential was measured as a water quality parameter in a raw material system 1, an oxidation-reduction potential was measured as a water quality parameter in a raw material system 2, an oxidation-reduction potential, turbidity, pH, and temperature were measured as water quality parameters in a papermaking system, and a foam height was measured as a water quality parameter in a recovery system, and average values at 24 hours prior to the production of a corresponding paper product were used. Fig. 7 is a schematic view of a facility which produces paper according to Example 1. In addition, grammage of the paper product was measured as a result parameter. Furthermore, the number of defects of the paper product was measured and 572 data sets of these pieces of data were prepared. Using 65% (372) of data sets in a former part in a chronological order among the data sets, a relational model between the number of defects that occur within 24 hours and a function of seven water quality parameters and one result parameter (hereinafter, also referred to as a "defect index $\alpha$") was created. More specifically, as a procedure of creating the defect index $\alpha$, after excluding parameters which are separated from an average value by two standard deviations or more as outliers, regression analysis was performed using SPSS Modeler manufactured by the International Business Machines Corporation. The correlation coefficient between the defect index $\alpha$ and the number of defects obtained by the regression analysis was 0.71 ($p < 0.05$), revealing a strong correlation.

Next, in order to validate a prediction accuracy of the defect index $\alpha$ with respect to the number of defects to occur in the future, the water quality parameters and the parameter were applied to the relational model described above to calculate the defect index α with respect to 35% (200) of data sets in a latter part in a chronological order among the data sets to calculate a correlation coefficient with the number of defects, which turned out to be 0.71 ($p < 0.05$). Accordingly, it was confirmed that there is a strong correlation between the number of defects and the defect index α and that the defect index α is effective in predicting the number of defects to occur in the future.

Fig. 8 shows plots of the number of defects of a total of 572 data sets against a defect index α according to Example 1. Note that in Fig. 8, 372 data sets for relational model creation are indicated by circles and 200 data sets for accuracy validation are indicated by squares.

Fig. 9 is a graph showing a magnitude of influence of each parameter with respect to the defect index α. When evaluating processing for reducing the number of defects, the number of defects can also be reduced in an efficient manner by identifying parameters with a large influence with respect to the defect index (proportional to the number of defects) and preferentially studying and improving a cause of a change in such parameters.

Example 2

In a production facility of cardboard-base paper (liner), pH and electrical conductivity were measured as water quality parameters in raw material systems 1 to 3, pH and oxidation-reduction potential were measured as water quality parameters in a raw material system 2, pH and electrical conductivity were measured as water quality parameters in a raw material system 3, and water temperature and electrical conductivity were measured as water quality parameters in a papermaking system (refer to Fig. 7). Furthermore, an original unit of usage of a paper strengthening agent of the produced paper product was measured at the same time as the measurement of the water quality parameters and 60 data sets of these pieces of data were prepared. The data sets were randomly divided by a ratio of 7:3 and 70% (42 sets) were used as data for relational model creation while 30% (18 sets) were used as data for model validation.

First, using the data for relational model creation, a relational model indicating a reciprocal of the original unit of usage of a paper strengthening agent was considered a "paper strength index" and was created as a function of the eight water quality parameters described above. More specifically, as a procedure of creating the paper strength index, after excluding parameters which are separated from an average value by two standard deviations or more as outliers, regression analysis was performed using SPSS Modeler manufactured by the International Business Machines Corporation. The correlation coefficient between the paper strength index and the original unit of usage of a paper strengthening agent obtained by the regression analysis was -0.58 ($p < 0.05$), revealing correlation.

Next, in order to validate the accuracy of the paper strength index, the paper strength index was calculated from the water quality parameters and a correlation coefficient with the original unit of usage of a paper strengthening agent was calculated with respect to the remaining 30% of data sets among the data sets, resulting in a value of -0.59 ($p < 0.05$) which confirms that there is correlation between the original unit of usage of a paper strengthening agent and the paper strength index.

Fig. 10 shows plots of an original unit of usage of a paper strengthening agent of the total of 60 data sets according to Example 2 against a paper strength index value. Note that in Fig. 10, 42 data sets for relational model creation are indicated by circles and 18 data sets for accuracy validation are indicated by squares.

Fig. 11 is a graph showing a magnitude of influence of each parameter with respect to the paper strength index. When evaluating stabilization of strength (paper strength) of a paper product or improvement of an original unit of usage of a paper strengthening agent, by identifying parameters with a large influence with respect to the paper strength index (proportional to the original unit of usage of a paper strengthening agent) and preferentially studying and improving a cause of a change in such parameters, product strength (paper strength) can be stabilized and the original unit of usage of a paper strengthening agent can be improved more efficiently.

Example 3

In a water system of a paperboard producing facility (a continuous water system made up of a raw material system, a papermaking system, and a recovery system), temperature, pH, an oxidation-reduction potential, electrical conductivity, turbidity, and standing-supernatant turbidity were measured as water quality parameters in a raw material system, pH, an oxidation-reduction potential, electrical conductivity, and turbidity were measured as water quality parameters in a papermaking system, and turbidity was measured as a water quality parameter in a recovery system (refer to Fig. 7). An operation start timing, a papermaking speed, an additive amount of internal chemicals, moisture content in felt, ash content in paper, product grammage, and product brand were used as control parameters. Furthermore, a paper breakage timing was measured and 138 and 276 data sets of these pieces of data were prepared. Note that as the water quality parameters, the control parameters, and the paper breakage timing, those of a same time were used. Using the data sets, a relational model between paper breakage that occurs within 24 hours and a function of the water quality parameters and the control parameters (hereinafter, also referred to as a "paper breakage index") was created in a similar manner to Example 1 and Example 2. More specifically, as a procedure of creating a paper breakage occurrence index, after excluding parameters which are separated from an average value by two standard deviations or more as outliers, regression analysis was performed using SPSS Modeler manufactured by the International Business Machines Corporation to create the relational model. Note that a plot diagram will omitted due to the huge number of data sets.

The water quality parameters and the control parameters were measured during actual operation (paperboard production) in the same water system of the paperboard producing facility and the paper breakage index was obtained using the relational model created as described above. In addition, the number of actual occurrences of paper breakage corresponding to the paper breakage index was also measured. Measurements were performed twice in total on different dates and at different times.

Fig. 12 and Fig. 13 are graphs showing a change over time of occurrences of paper breakage and a paper breakage index. In Fig. 12 and Fig. 13, an axis of ordinate represents occurrences of paper breakage and the paper breakage index and an axis of abscissa represents time. A circle indicates a presence or absence of an actual occurrence of paper breakage (0 when paper breakage does not occur and 1 when paper breakage occurs) (left of axis of ordinate) and a square indicates the paper breakage index (right of axis of ordinate). Fig. 12 and Fig. 13 clearly show that paper breakage actually occurs when the paper breakage index approaches 1. It was also shown that paper breakage can be predicted by appropriately providing a threshold with respect to the paper breakage index.

Example 4

In a water system of a paper producing facility (a continuous water system made up of a raw material system, a papermaking system, a recovery system, and a drainage system), pH and turbidity were measured as water quality parameters in a raw material system 1, an oxidation-reduction potential was measured as a water quality parameter in a papermaking system, and electrical conductivity was measured as a water quality parameter in a drainage system, and measured values at 16 hours prior to the production of a corresponding paper product were used. Fig. 14 is a schematic view of a facility which produces paper according to Example 4. In addition, a papermaking speed of the paper product was measured as a control parameter. Furthermore, grammage of the paper product was measured as a result parameter. Moreover, the number of defects of the paper product was measured and 647 data sets of these pieces of data were prepared. Using the data sets, a relational model between the number of defects and a function of the four water quality parameters, the one control parameter, and the one result parameter (hereinafter, also referred to as a "defect index (3") was created. More specifically, as a procedure of creating the defect index β, after excluding parameters which are separated from an average value by two standard deviations or more as outliers, an analysis by a state space model was performed using a package KFAS of R language that is a programming language for statistical analysis. The correlation coefficient between the defect index β and the number of defects obtained by the state space model was 0.62 ($p < 0.05$), revealing a correlation.

In order to validate a prediction accuracy of the defect index β with respect to the number of defects to occur in the future, an analysis for calculating the defect index β up to 16 hours in the future was repetitively and sequentially performed in one batch units at one hour intervals for a total of 16 batches (16 times) using latest 647 data sets acquired every 16 hours to obtain 255 sets of the defect index β. The production of paper was stopped between hours 172 to 264. A calculation of the correlation coefficient between the defect indices β and the number of defects that actually occurred up to 16 hours in the future resulted in a value of 0.80 ($p < 0.05$). Accordingly, it was confirmed that there is a strong correlation between the number of defects and the defect index β and that the defect index β is effective in predicting the number of defects to occur in the future.

Fig. 15 shows plots of the number of defects of a total of 647 data sets for data creation according to Example 4 against values of the defect index β. In addition, Fig. 16 shows plots of the number of defects of a total of 255 data sets for accuracy validation according to Example 4 against values of the defect index β for data creation.

Fig. 17 shows plots indicating a change over time of a defect index calculated from the total of 255 data sets for accuracy validation according to Example 4.

Fig. 18 is a graph showing a magnitude of influence of each parameter with respect to the defect index β. When evaluating processing for reducing the number of defects, the number of defects can also be reduced in an efficient manner by identifying parameters with a large influence with respect to the defect index β (proportional to the number of defects) and preferentially studying and improving a cause of a change in such parameters.

Example 5

In a water system of a paper producing facility (a continuous water system made up of a raw material system, a papermaking system, a recovery system, and a drainage system), pH and turbidity were measured and used as water quality parameters in a raw material system, an oxidation-reduction potential was measured and used as a water quality parameter in a papermaking system, and electrical conductivity was measured and used as a water quality parameter in a drainage system (refer to Fig. 14). In addition, a papermaking speed of the paper product was measured as a control parameter. Furthermore, grammage of the paper product was measured as a result parameter. Moreover, the number of defects of the paper product was measured and 631 data sets of these pieces of data were prepared. Using the data sets, a relational model between the number of defects and a function of the four water quality parameters, the one control parameter, and the one result parameter (hereinafter, also referred to as a "defect index γ") was created. More specifically, as a procedure of creating the defect index γ, after excluding parameters which are separated from an average value by two standard deviations or more as outliers, an analysis by a VAR model that is a type of time-series analysis was performed using a package vars of R language that is a programming language for statistical analysis. It was confirmed that the defect index γ obtained by the VAR model and the number of defects that actually occurred are linked to each other.

In order to validate a prediction accuracy of the defect index γ with respect to the number of defects to occur in the future, an analysis for calculating the defect index γ up to 6 hours in the future was repetitively and sequentially performed in one batch units at one hour intervals for a total of 46 batches (46 times) using latest 631 data sets acquired every 6 hours to obtain 271 sets of the defect index γ. A calculation of the correlation coefficient between the defect indices γ and the number of defects that actually occurred up to 6 hours in the future resulted in a value of 0.64 ($p < 0.05$). Accordingly, it was confirmed that there is a correlation between the number of defects and the defect index γ and that the defect index γ is effective in predicting the number of defects to occur in the future.

Fig. 19 shows plots of the number of defects of a total of 631 data sets for relational model creation against values of a defect index γ. Fig. 20 shows plots of the number of defects of a total of 255 data sets for accuracy validation according to Example 5 against the values of the defect index γ.

Fig. 21 shows plots indicating a change over time of a defect index calculated from the total of 271 data sets for accuracy validation according to Example 5.

Fig. 22 is a graph showing a magnitude of influence of each parameter with respect to the defect index γ. Note that in Fig. 22, only parameters that are significant ($p < 0.10$) with respect to a significance level of 10% are shown. When evaluating processing for reducing the number of defects, the number of defects can also be reduced in an efficient manner by identifying parameters with a large influence with respect to the defect index γ (proportional to the number of defects) and preferentially studying and improving a cause of a change in such parameters.

Example 6

In a water system of a paper producing facility (a continuous water system made up of a raw material system, a papermaking system, and a drainage system), pH and turbidity were measured as water quality parameters in a raw material system, an oxidation-reduction potential was measured as a water quality parameter in a papermaking system, and electrical conductivity was measured as a water quality parameter in a drainage system, and numerical values at 16 hours prior to the production of a corresponding paper product were used (refer to Fig. 14). In addition, a papermaking speed of the paper product was measured as a control parameter. Furthermore, grammage of the paper product and the number of defects of the paper product were measured as result parameters, and 1706 data sets of these pieces of data were prepared. Using 71% (1216) of data sets in a former part in a chronological order among the data sets, a relational model between the number of defects that occur 16 hours in the future and a function of the four water quality parameters, the one control parameter, and the one result parameter (hereinafter, also referred to as a "defect index δ") was created. More specifically, as a procedure of creating the defect index δ, after excluding parameters which are separated from an average value by two standard deviations or more as outliers, an analysis by a multilayer perceptron that is a type of a neural network was performed using SPSS Modeler manufactured by the International Business Machines Corporation. The correlation coefficient between the defect index δ and the number of defects obtained by the multilayer perceptron was 0.73 ($p < 0.05$), revealing a strong correlation.

Next, in order to validate a prediction accuracy of the defect index δ with respect to the number of defects to occur in the future, the water quality parameters and the parameters were applied to the relational model described above to calculate the defect index δ with respect to 29% (490) of data sets in a latter part in a chronological order among the data sets to calculate a correlation coefficient with the number of defects, which turned out to be 0.73 ($p < 0.05$). Accordingly, it was confirmed that there is a strong correlation between the number of defects and the defect index δ and that the defect index δ is effective in predicting the number of defects to occur in the future.

Fig. 23 shows plots of the number of defects of a total of 1216 data sets for relational model creation according to Example 6 against values of the defect index δ. Fig. 24 shows plots of the number of defects of a total of 490 data sets for accuracy validation according to Example 6 against the values of the defect index δ. Plots of the number of defects of the data sets according to Example 6 against values of the defect index δ.

Fig. 25 is a graph showing a magnitude of influence of each parameter with respect to the defect index δ. When evaluating processing for reducing the number of defects, the number of defects can also be reduced in an efficient manner by identifying parameters with a large influence with respect to the defect index δ (proportional to the number of defects) and preferentially studying and improving a cause of a change in such parameters.

Example 7

In a water system of a paper producing facility (a continuous water system made up of a raw material system, a papermaking system, and a drainage system), pH and turbidity were measured as water quality parameters in a raw material system, an oxidation-reduction potential was measured as a water quality parameter in a papermaking system, and electrical conductivity was measured as a water quality parameter in a drainage system, and numerical values at 16 hours prior to the production of a corresponding paper product were used (refer to Fig. 14). In addition, a flow rate of a headbox and a papermaking speed of the paper product were measured as control parameters. Furthermore, grammage of the paper product and the number of defects of the paper product were measured as result parameters, and 2040 data sets of these pieces of data were prepared. Using 74% (1503) of data sets in a former part in a chronological order among the data sets, a relational model between the number of defects that occur 16 hours in the future and a function of the four water quality parameters, the two control parameters, and the one result parameter (hereinafter, also referred to as a "defect index ε") was created. More specifically, as a procedure of creating the defect index ε, after excluding parameters which are separated from an average value by two standard deviations or more as outliers, an analysis by XGBoost being a type of a decision tree and a type of ensemble learning was performed using SPSS Modeler manufactured by the International Business Machines Corporation. The correlation coefficient between the defect index ε and the number of defects obtained by XGBoost was 0.95 ($p < 0.05$), revealing a strong correlation.

Next, in order to validate a prediction accuracy of the defect index ε with respect to the number of defects to occur in the future, the water quality parameters and the parameters were applied to the relational model described above to calculate the defect index ε with respect to 26% (537) of data sets in a latter part in a chronological order among the data sets to calculate a correlation coefficient with the number of defects, which turned out to be 0.57 ($p < 0.05$). Accordingly, it was confirmed that there is a correlation between the number of defects and the defect index ε and that the defect index ε is effective in predicting the number of defects to occur in the future.

Fig. 26 shows plots of the number of defects of a total of 1503 data sets for relational model creation according to Example 7 against the defect index ε. Fig. 27 shows plots of the number of defects of a total of 537 data sets for accuracy validation according to Example 7 against the defect index ε.

Fig. 28 is a graph showing a magnitude of influence of each parameter with respect to the defect index ε. When evaluating processing for reducing the number of defects, the number of defects can also be reduced in an efficient manner by identifying parameters with a large influence with respect to the defect index ε (proportional to the number of defects) and preferentially studying and improving a cause of a change in such parameters.

Reference Signs List

1 inference system
2 inference apparatus
3 output apparatus or output unit
4 parameter information measurement apparatus or parameter information measuring unit
21 parameter information acquiring unit
22 relational model information acquiring unit or first relational model information acquiring unit
23 inferring unit or first inferring unit
24 second inferring unit
25 relational model information creating unit or first relational model information creating unit
26 second relational model creating unit
27 second relational model information acquiring unit
51 communicating unit
52 storage unit
53 control unit
54 communication bus

## Claims

1. An inference apparatus for inferring a potential result which may arise in a water system or which may be derived from the water system and may arise in future, the inference apparatus comprising:

a parameter information acquiring unit; a relational model information acquiring unit; and an inferring unit, wherein the parameter information acquiring unit acquires parameter information which includes two or more of one type of parameter among: a water quality parameter related to water quality of the water system; a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system, the relational model information acquiring unit acquires relational model information which is created in advance and which indicates a relationship between the potential result or an index related to the potential result and the two or more parameters, and

the inferring unit infers the potential result or the index related to the potential result based on the parameter information and the relational model information.

**2.** The inference apparatus according to claim 1, wherein
the relational model is a model obtained from a regression analysis, a time-series analysis, a decision tree, a neural network, Bayes, clustering, or ensemble learning between a prior confirmation result corresponding to the potential result or an index related to the prior confirmation result and the two or more parameters.

**3.** The inference apparatus according to claim 1 or 2, wherein
the water system is a water system in a process of producing a paper product.

**4.** The inference apparatus according to claim 3, wherein
the water quality parameter is one or more parameters selected from the group consisting of pH, electrical conductivity, an oxidation-reduction potential, a zeta potential, turbidity, temperature, a foam height, a biochemical oxygen demand (BOD), a chemical oxygen demand (COD), absorbance, color, a particle size distribution, an agglomeration degree, an amount of foreign matter, a foaming area on a water surface, an area of underwater contamination, an amount of bubbles, an amount of glucose, an amount of organic acids, an amount of starch, an amount of calcium, an amount of total chlorine, an amount of free chlorine, an amount of dissolved oxygen, a cationic demand, an amount of hydrogen sulfide, an amount of hydrogen peroxide, and a respiration rate of microorganisms in the water system.

**5.** The inference apparatus according to claim 3 or 4, wherein
the control parameter is one or more parameters selected from the group consisting of an operating speed (a papermaking speed) of a paper machine, a rotational speed of a filter cloth of a raw material dehydrator, a rotational speed of a filter cloth of a cleaning machine, an additive amount of chemicals to the water system, an additive amount of chemicals relative to raw materials to be added to the water system, an additive amount of chemicals relative to facilities related to the water system, an amount of vapor for heating, temperature of vapor for heating, pressure of vapor for heating, a flow rate from a headbox, nip pressure of a press part, felt vacuum pressure of a press part, a blending ratio of papermaking raw material, a blended amount of waste sheets of papermaking raw material, a mesh size of a screen for papermaking raw material, a gap distance between a rotor and a stator of a beater, freeness, and a degree of beating.

**6.** The inference apparatus according to any one of claims 3 to 5, wherein
the result parameter is one or more parameters selected from the group consisting of a unit weight (grammage) of the paper product, a yield, white water concentration, moisture content of the paper product, an amount of vapor in a facility producing the paper product, temperature of vapor in a facility producing the paper product, pressure of vapor in a facility producing the paper product, thickness of paper product, concentration of ash in the paper product, a type of defect of the paper product, the number of defects in the paper product, a timing of paper breakage in a process, freeness, a degree of beating, and an amount of aeration.

**7.** An inference system for inferring a potential result which may arise in a water system or which may be derived from the water system and may arise in future, the inference system comprising:

a parameter information acquiring unit; a relational model information acquiring unit; and an inferring unit, wherein the parameter information acquiring unit acquires parameter information which includes two or more of one type of parameter among: a water quality parameter related to water quality of the water system; a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system,
the relational model information acquiring unit acquires relational model information which is created in advance and which indicates a relationship between the potential result or an index related to the potential result and the two or more parameters, and
the inferring unit infers the potential result or the index related to the potential result based on the parameter information and the relational model information.

**8.** An inference program for inferring a potential result which may arise in a water system or which may be derived from the water system and may arise in future, the inference program causing:

a computer to function as: a water quality information acquiring unit; a relational model information acquiring unit; and an inferring unit, wherein
the parameter information acquiring unit acquires parameter information which includes two or more of one type of parameter among: a water quality parameter related to water quality of the water system; a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system,
the relational model information acquiring unit acquires relational model information which is created in advance and which indicates a relationship between the potential result or an index related to the potential result and the two or more parameters, and
the inferring unit infers the potential result or the index related to the potential result based on the parameter information and the relational model information.

**9.** An inference method for inferring a potential result which may arise in a water system or which may be derived from the water system and may arise in future, the inference method comprising:

a parameter information acquiring step; a relational model information acquiring step; and an inferring step, wherein
in the parameter information acquiring step, parameter information which includes two or more of one type of parameter among: a water quality parameter related to water quality of the water system, a control parameter related to the water system, a facility related to the water system, or a control condition of a raw material to be added to the water system; and a result parameter which is a parameter having a different meaning from the potential result and which is related to a result having arisen in the water system, a facility related to the water system, or a raw material to be added to the water system or a result having been derived and arisen from the water system, a facility related to the water system, or a raw material to be added to the water system is acquired,
in the relational model information acquiring step, relational model information which is created in advance and which indicates a relationship between the potential result or an index related to the potential result and the two or more parameters is acquired, and
in the inferring step, the potential result or the index related to the potential result is inferred based on the parameter information and the relational model information.

Fig. 1

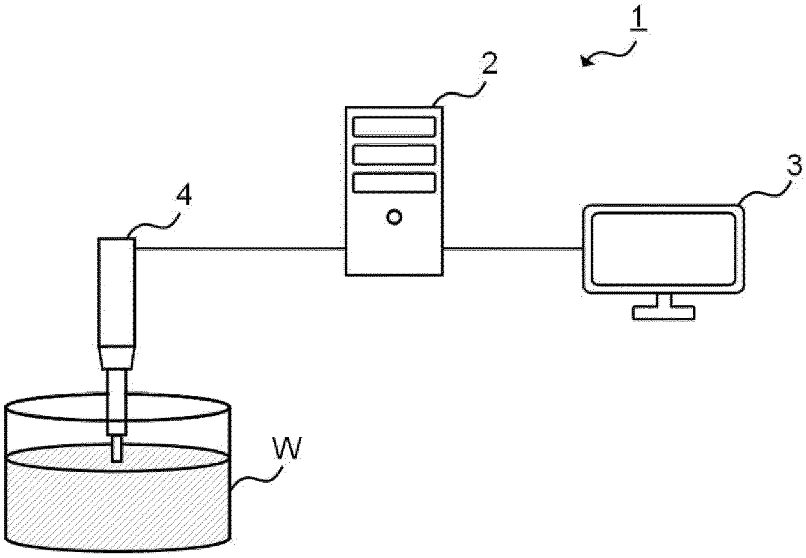
1
2
3
4
W

Fig. 2

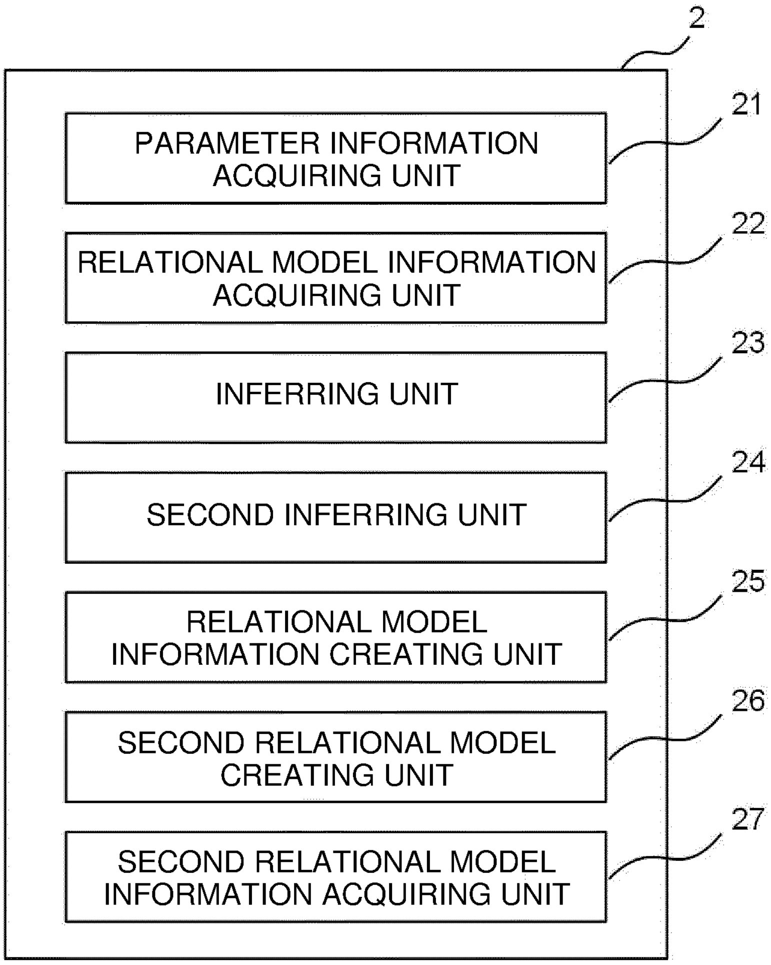
2
PARAMETER INFORMATION ACQUIRING UNIT
21
RELATIONAL MODEL INFORMATION ACQUIRING UNIT
22
INFERRING UNIT
23
SECOND INFERRING UNIT
24
RELATIONAL MODEL INFORMATION CREATING UNIT
25
SECOND RELATIONAL MODEL CREATING UNIT
26
SECOND RELATIONAL MODEL INFORMATION ACQUIRING UNIT
27

Fig. 3

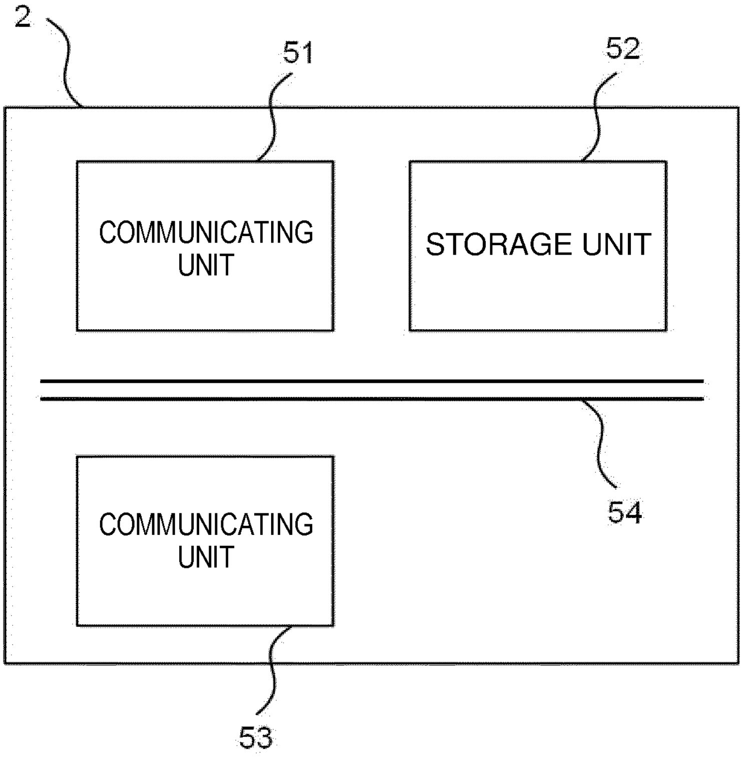
2
51
52
COMMUNICATING UNIT
STORAGE UNIT
54
COMMUNICATING UNIT
53

Fig. 4

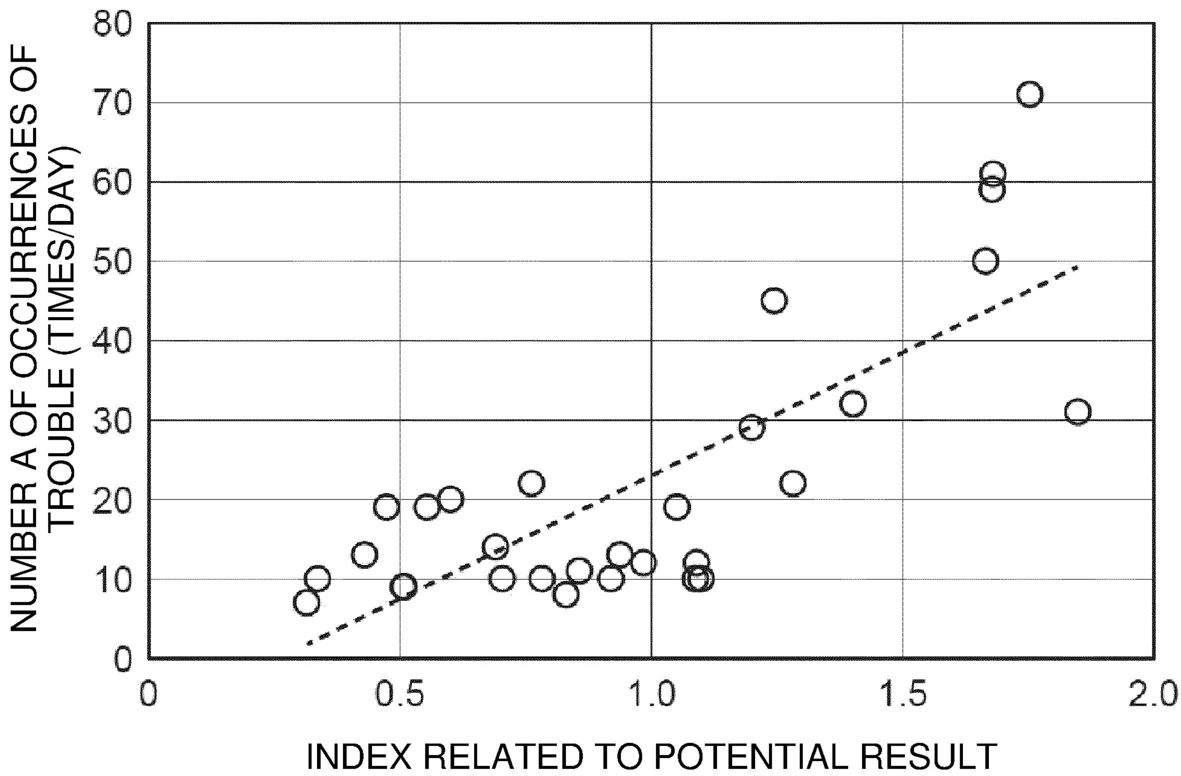
NUMBER A OF OCCURRENCES OF TROUBLE (TIMES/DAY)
80
70
60
50
40
30
20
10
0
0
0.5
1.0
1.5
2.0
INDEX RELATED TO POTENTIAL RESULT

Fig. 5

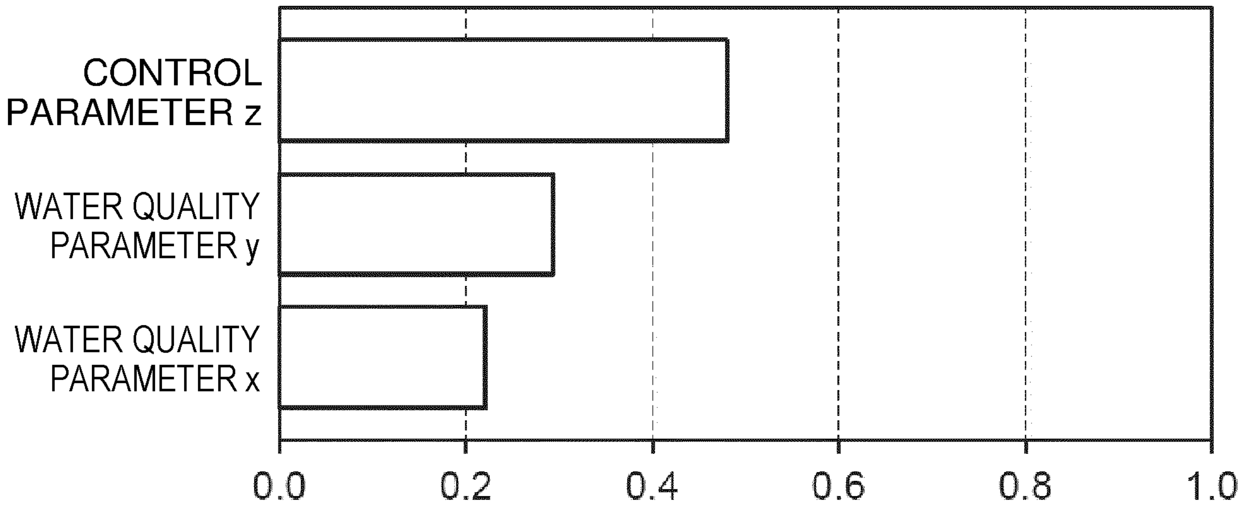
CONTROL PARAMETER z
WATER QUALITY PARAMETER y
WATER QUALITY PARAMETER x
0.0
0.2
0.4
0.6
0.8
1.0

Fig. 6

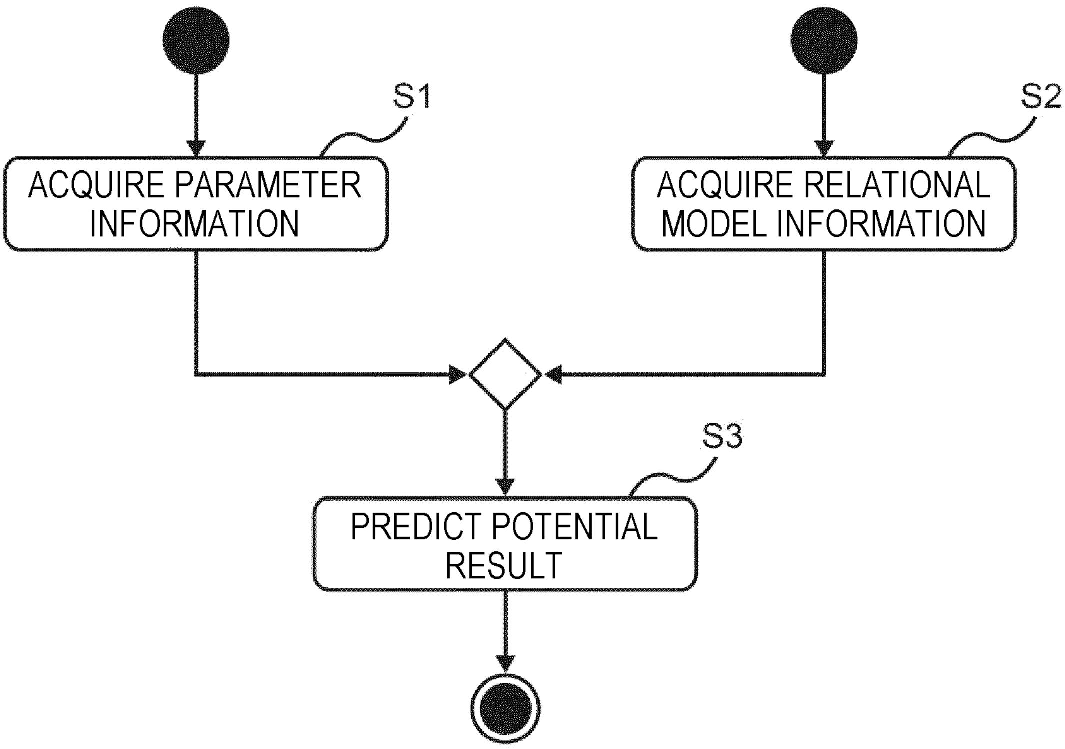
S1
ACQUIRE PARAMETER INFORMATION
S2
ACQUIRE RELATIONAL MODEL INFORMATION
S3
PREDICT POTENTIAL RESULT

Fig. 7

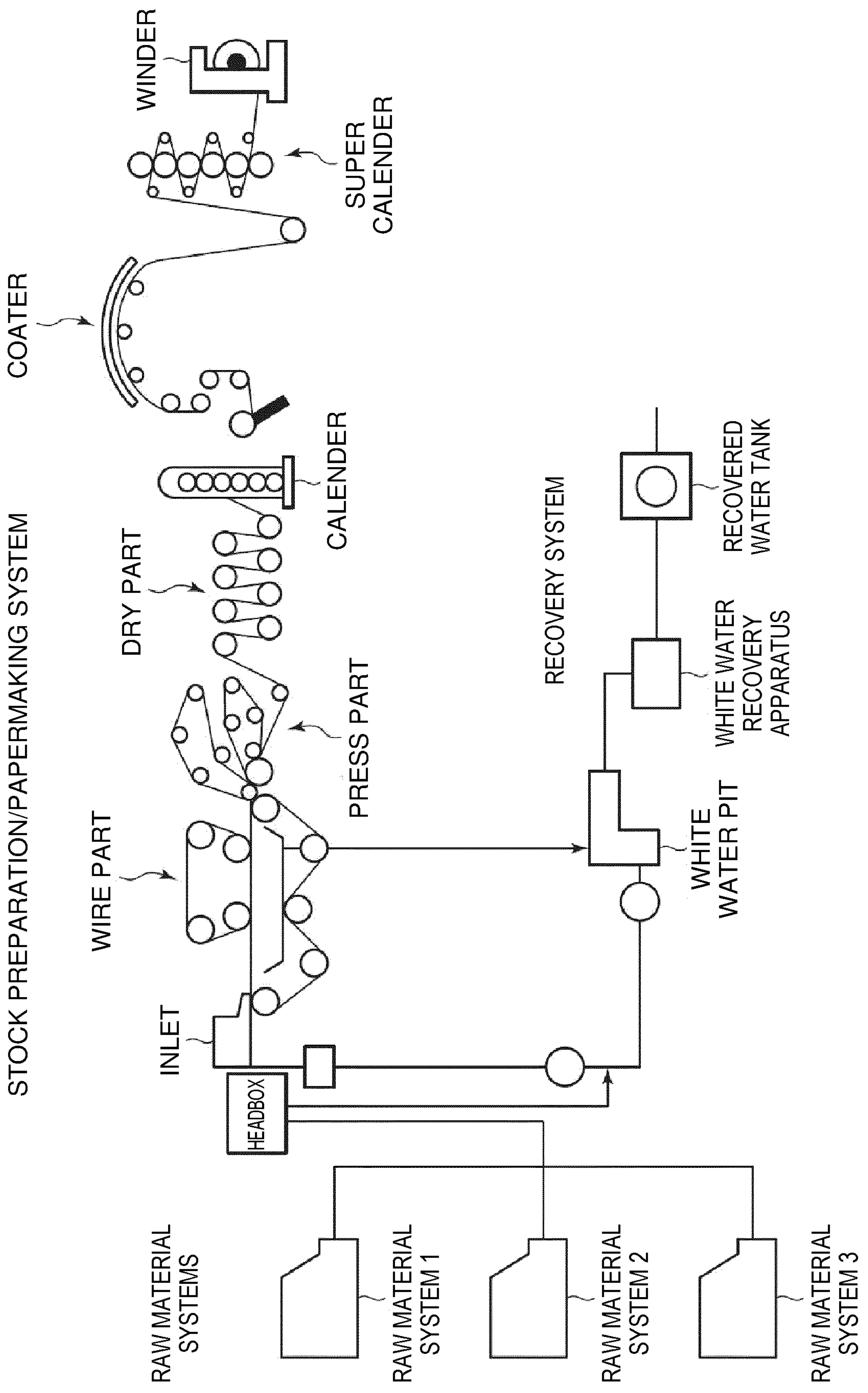
STOCK PREPARATION/PAPERMAKING SYSTEM
WINDER
SUPER CALENDER
COATER
CALENDER
DRY PART
PRESS PART
WIRE PART
INLET
HEADBOX
RECOVERY SYSTEM
RECOVERED WATER TANK
WHITE WATER RECOVERY APPARATUS
WHITE WATER PIT
RAW MATERIAL SYSTEMS
RAW MATERIAL SYSTEM 1
RAW MATERIAL SYSTEM 2
RAW MATERIAL SYSTEM 3

Fig. 8

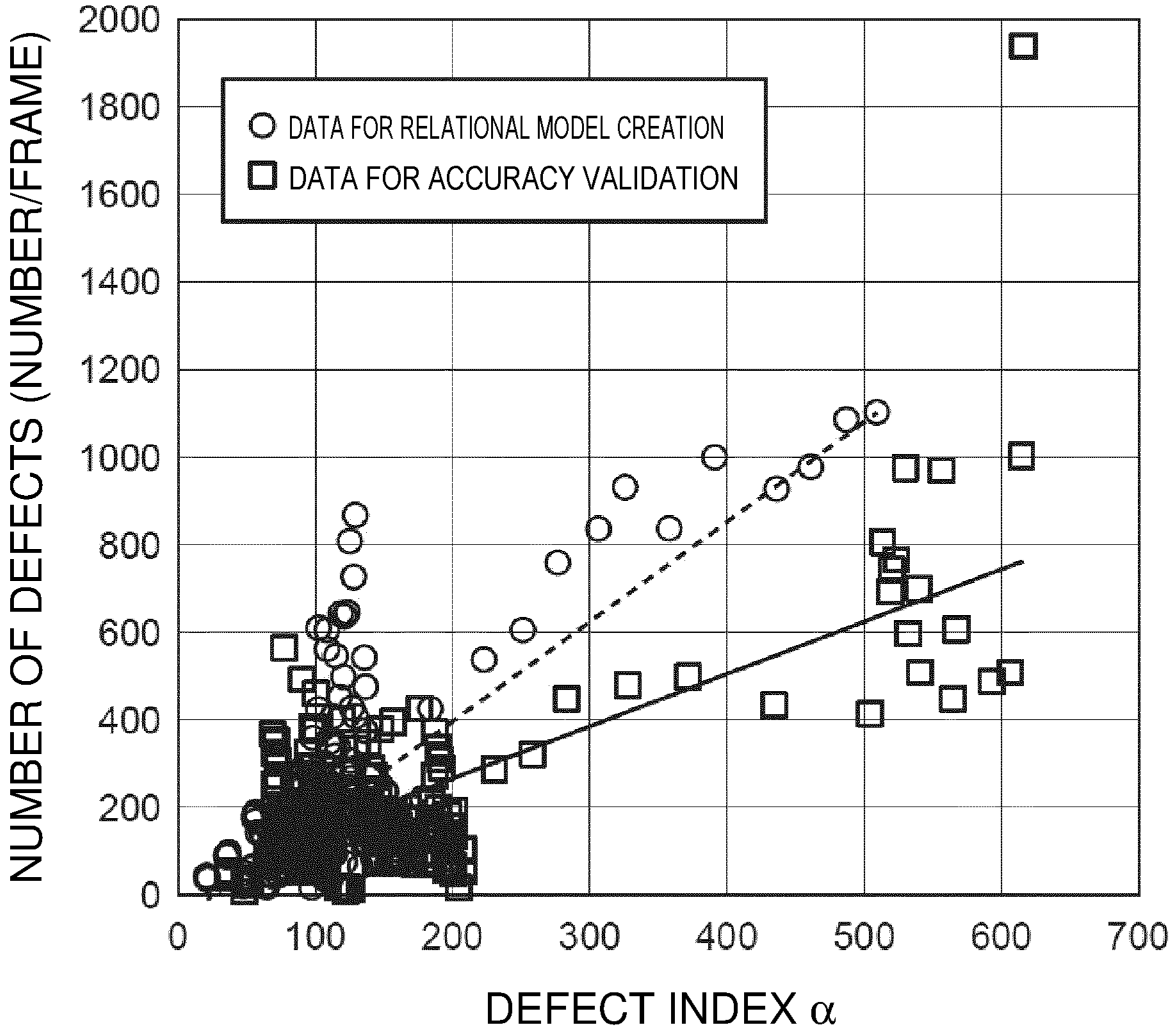
NUMBER OF DEFECTS (NUMBER/FRAME)
2000
1800
1600
1400
1200
1000
800
600
400
200
0
0
100
200
300
400
500
600
700
DEFECT INDEX α
DATA FOR RELATIONAL MODEL CREATION
DATA FOR ACCURACY VALIDATION

Fig. 9

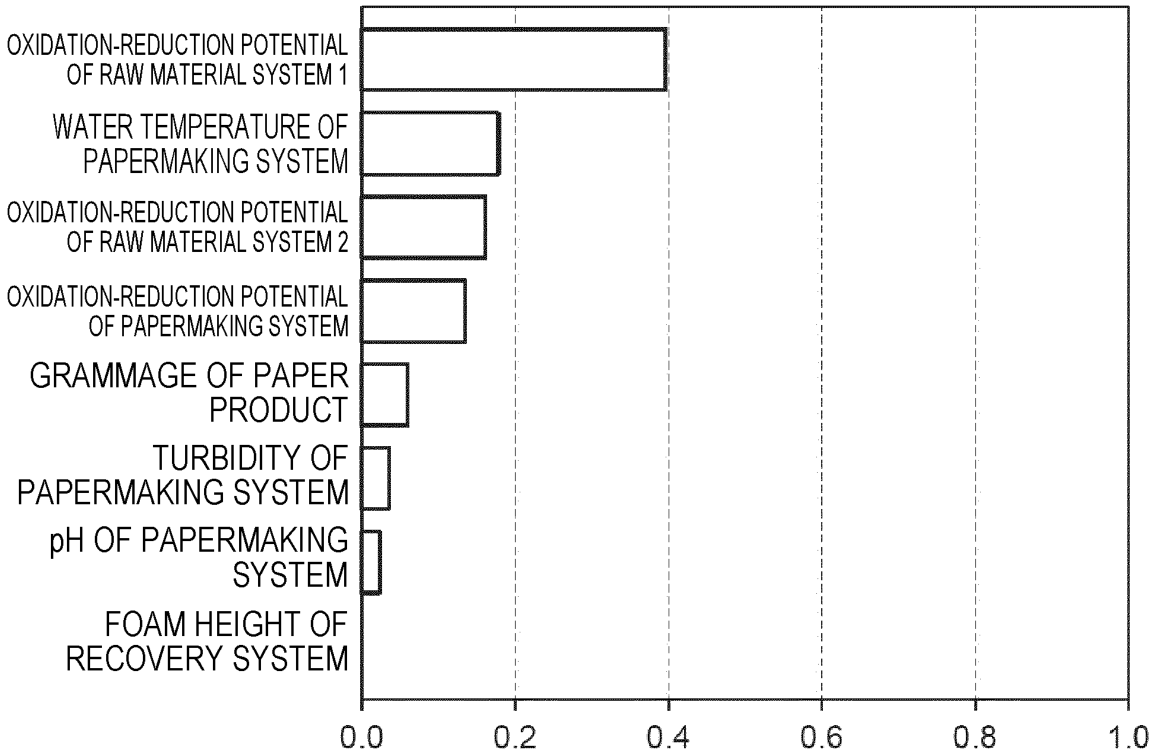
OXIDATION-REDUCTION POTENTIAL OF RAW MATERIAL SYSTEM 1
WATER TEMPERATURE OF PAPERMAKING SYSTEM
OXIDATION-REDUCTION POTENTIAL OF RAW MATERIAL SYSTEM 2
OXIDATION-REDUCTION POTENTIAL OF PAPERMAKING SYSTEM
GRAMMAGE OF PAPER PRODUCT
TURBIDITY OF PAPERMAKING SYSTEM
pH OF PAPERMAKING SYSTEM
FOAM HEIGHT OF RECOVERY SYSTEM
0.0
0.2
0.4
0.6
0.8
1.0

Fig. 10

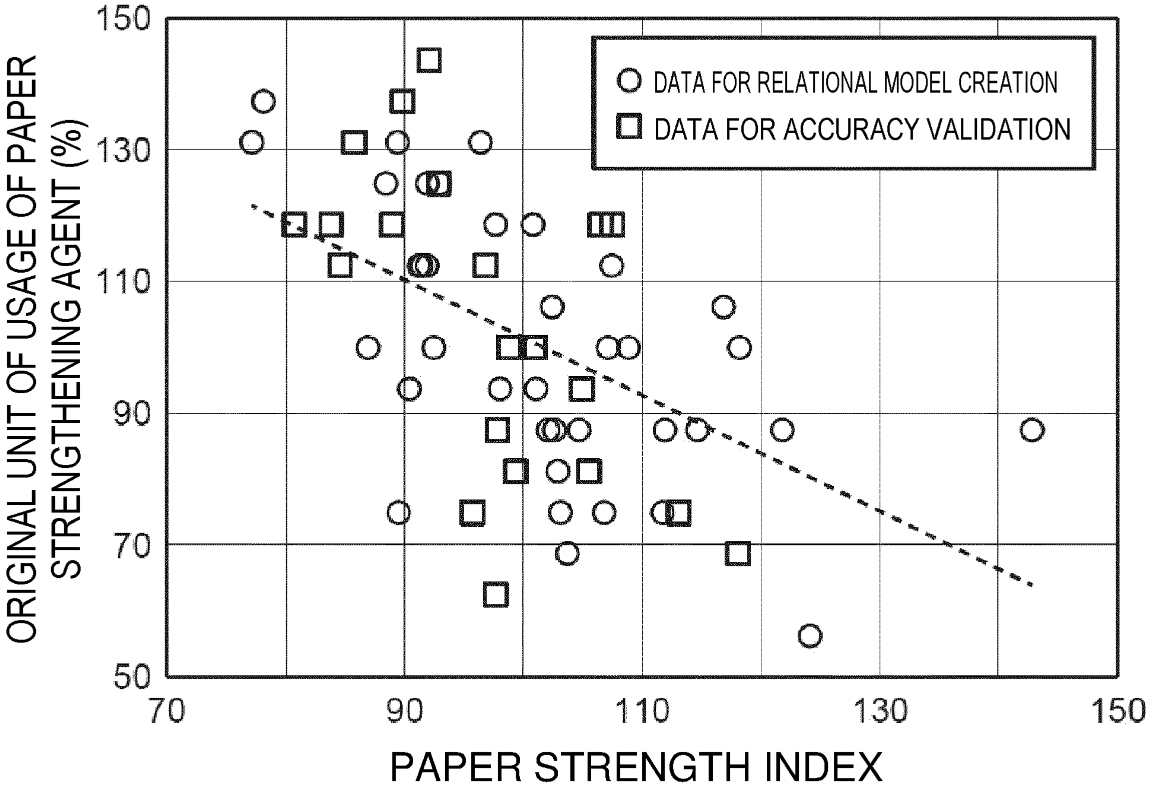
DATA FOR RELATIONAL MODEL CREATION
DATA FOR ACCURACY VALIDATION
ORIGINAL UNIT OF USAGE OF PAPER STRENGTHENING AGENT (%)
150
130
110
90
70
50
70
90
110
130
150
PAPER STRENGTH INDEX

Fig. 11

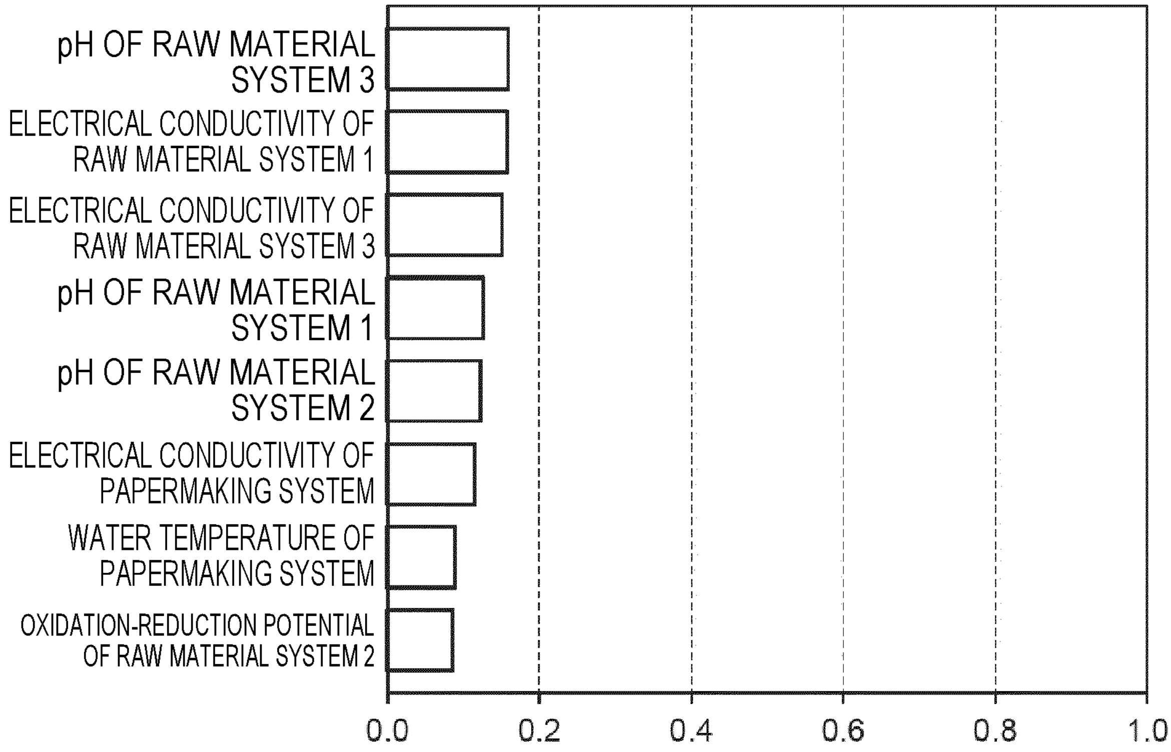
pH OF RAW MATERIAL SYSTEM 3
ELECTRICAL CONDUCTIVITY OF RAW MATERIAL SYSTEM 1
ELECTRICAL CONDUCTIVITY OF RAW MATERIAL SYSTEM 3
pH OF RAW MATERIAL SYSTEM 1
pH OF RAW MATERIAL SYSTEM 2
ELECTRICAL CONDUCTIVITY OF PAPERMAKING SYSTEM
WATER TEMPERATURE OF PAPERMAKING SYSTEM
OXIDATION-REDUCTION POTENTIAL OF RAW MATERIAL SYSTEM 2
0.0
0.2
0.4
0.6
0.8
1.0

Fig. 12

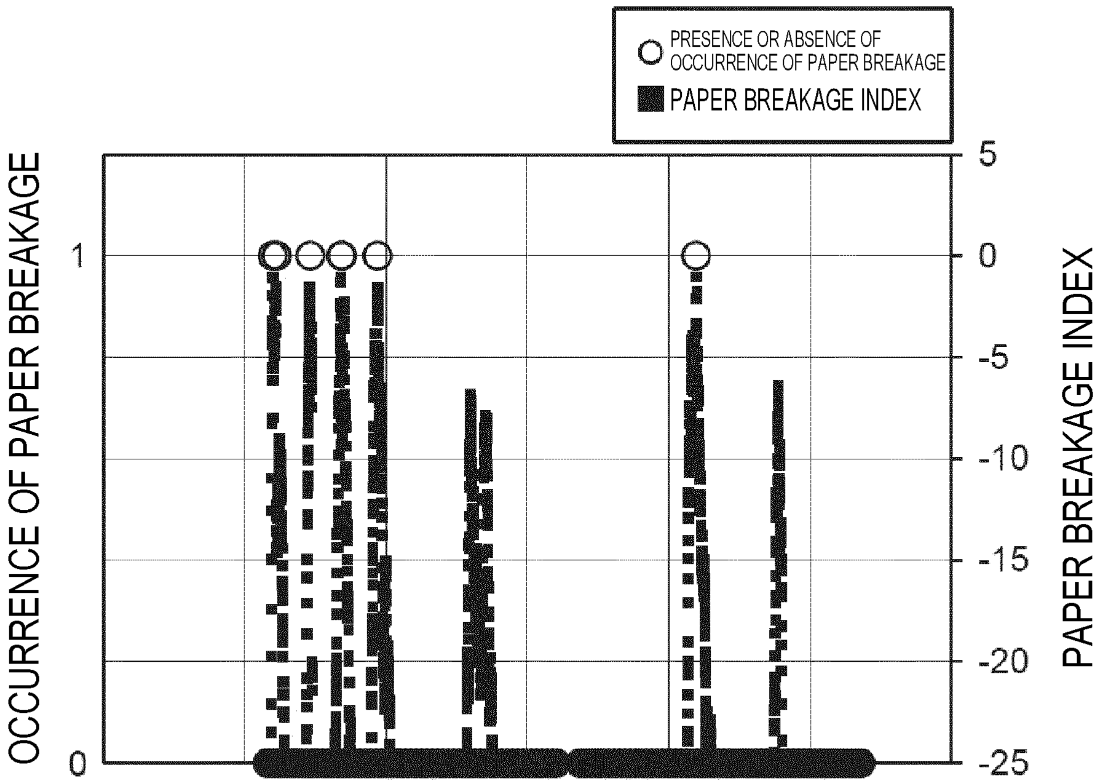
PRESENCE OR ABSENCE OF OCCURRENCE OF PAPER BREAKAGE
PAPER BREAKAGE INDEX
OCCURRENCE OF PAPER BREAKAGE
1
0
PAPER BREAKAGE INDEX
5
0
-5
-10
-15
-20
-25

Fig. 13

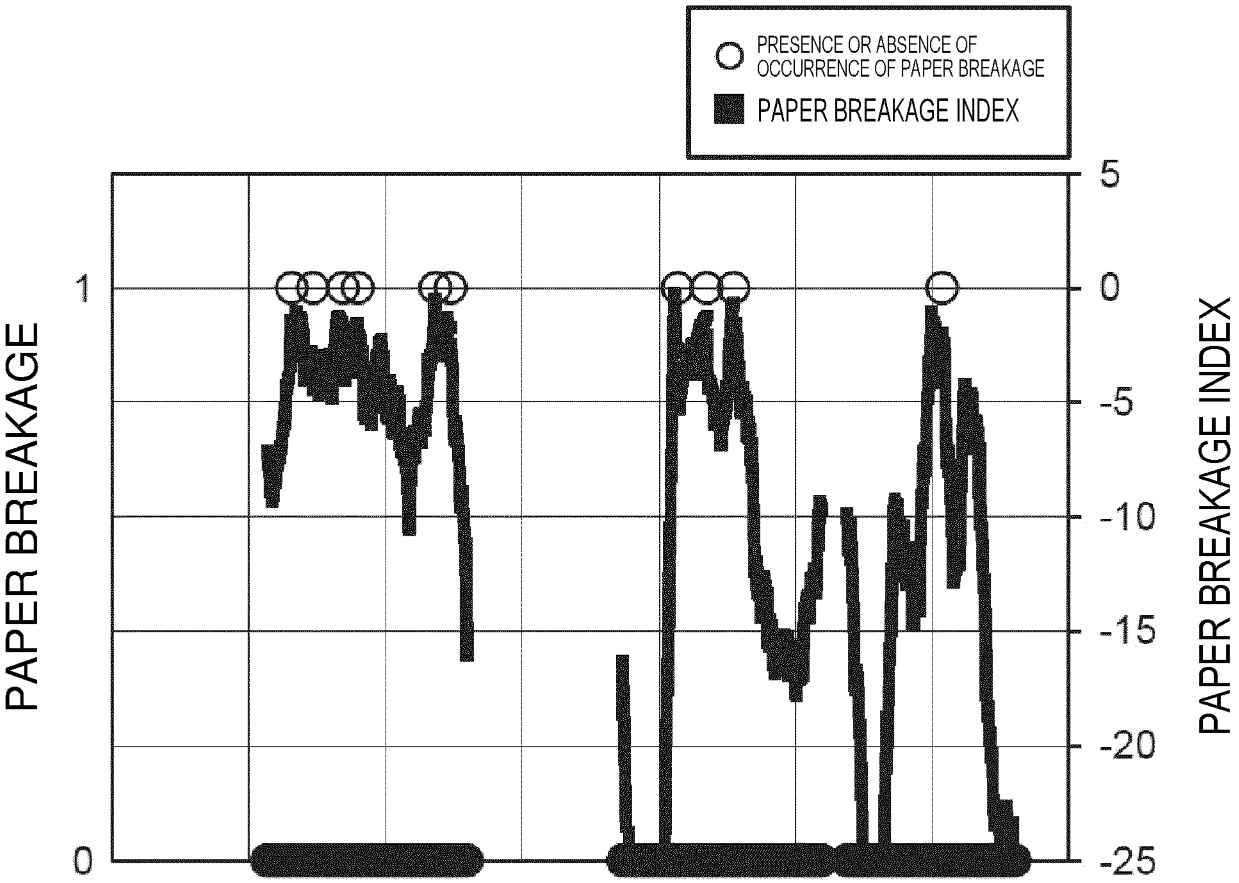
PRESENCE OR ABSENCE OF OCCURRENCE OF PAPER BREAKAGE
PAPER BREAKAGE INDEX
PAPER BREAKAGE
1
0
PAPER BREAKAGE INDEX
5
0
-5
-10
-15
-20
-25

Fig. 14

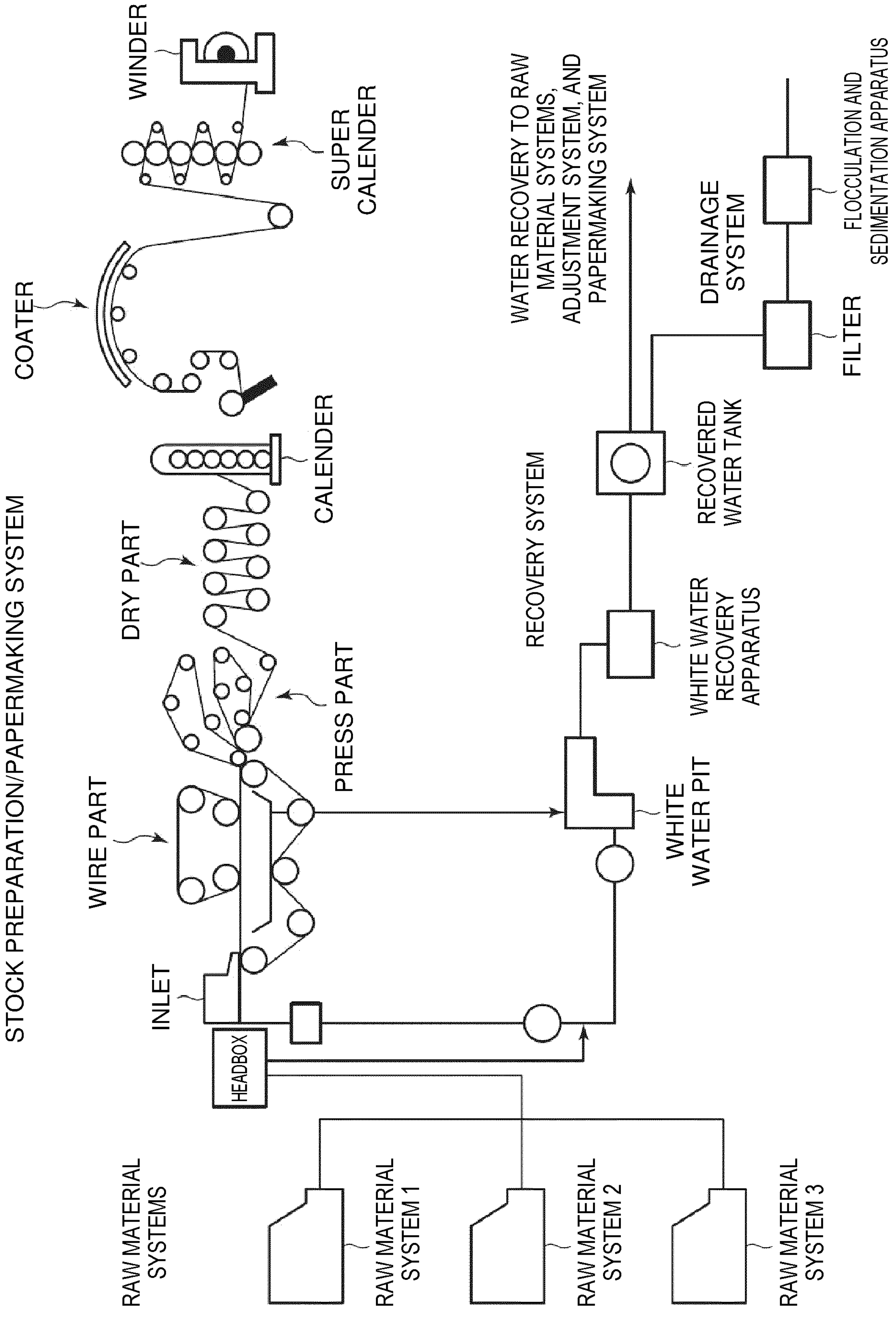
STOCK PREPARATION/PAPERMAKING SYSTEM
WINDER
SUPER CALENDER
COATER
CALENDER
DRY PART
PRESS PART
WIRE PART
INLET
HEADBOX
RAW MATERIAL SYSTEMS
RAW MATERIAL SYSTEM 1
RAW MATERIAL SYSTEM 2
RAW MATERIAL SYSTEM 3
RECOVERY SYSTEM
WATER RECOVERY TO RAW MATERIAL SYSTEMS, ADJUSTMENT SYSTEM, AND PAPERMAKING SYSTEM
DRAINAGE SYSTEM
FLOCCULATION AND SEDIMENTATION APPARATUS
FILTER
RECOVERED WATER TANK
WHITE WATER RECOVERY APPARATUS
WHITE WATER PIT

Fig. 15

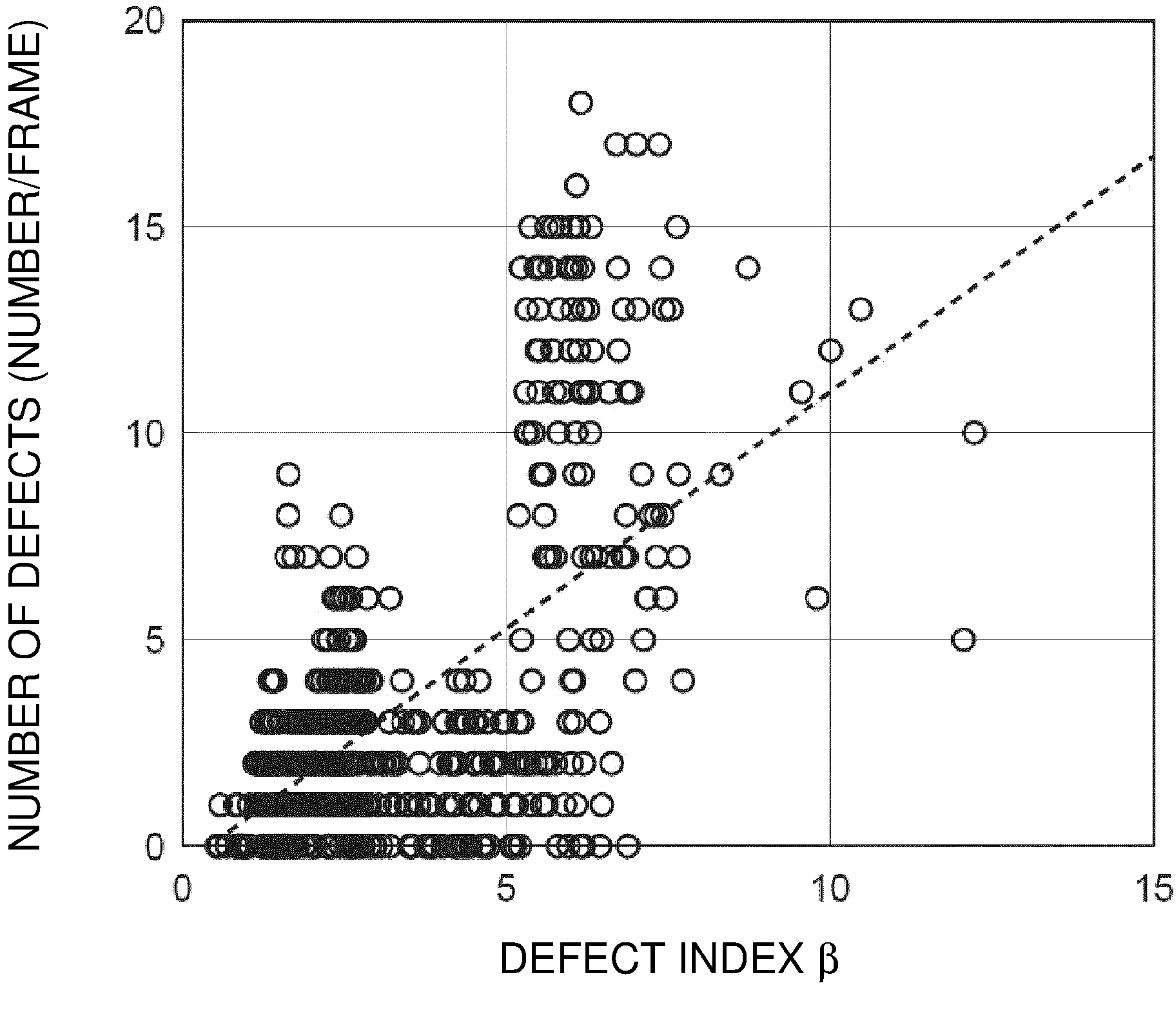
NUMBER OF DEFECTS (NUMBER/FRAME)
20
15
10
5
0
0
5
10
15
DEFECT INDEX β

Fig. 16

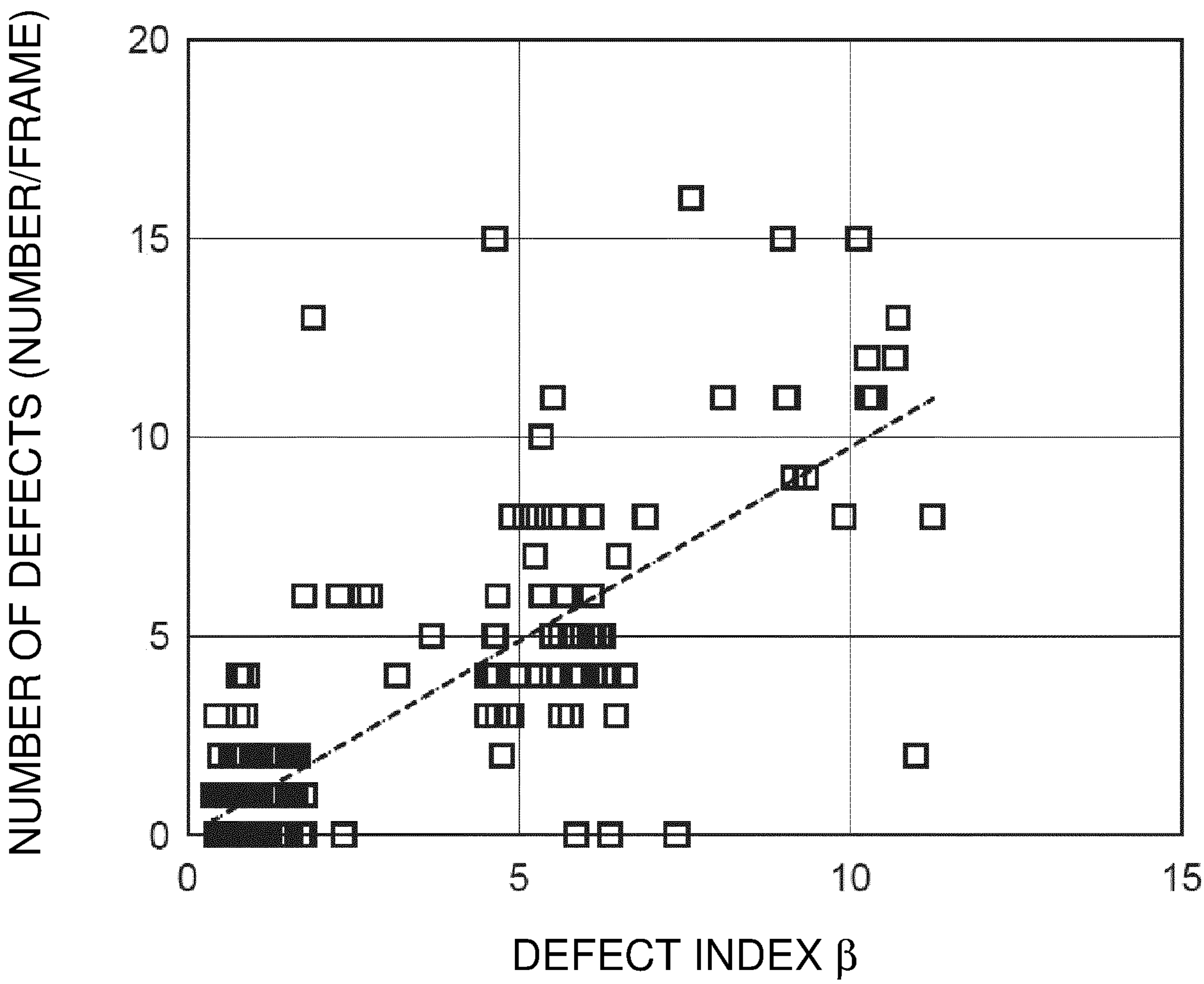
NUMBER OF DEFECTS (NUMBER/FRAME)
20
15
10
5
0
0
5
10
15
DEFECT INDEX β

Fig. 17

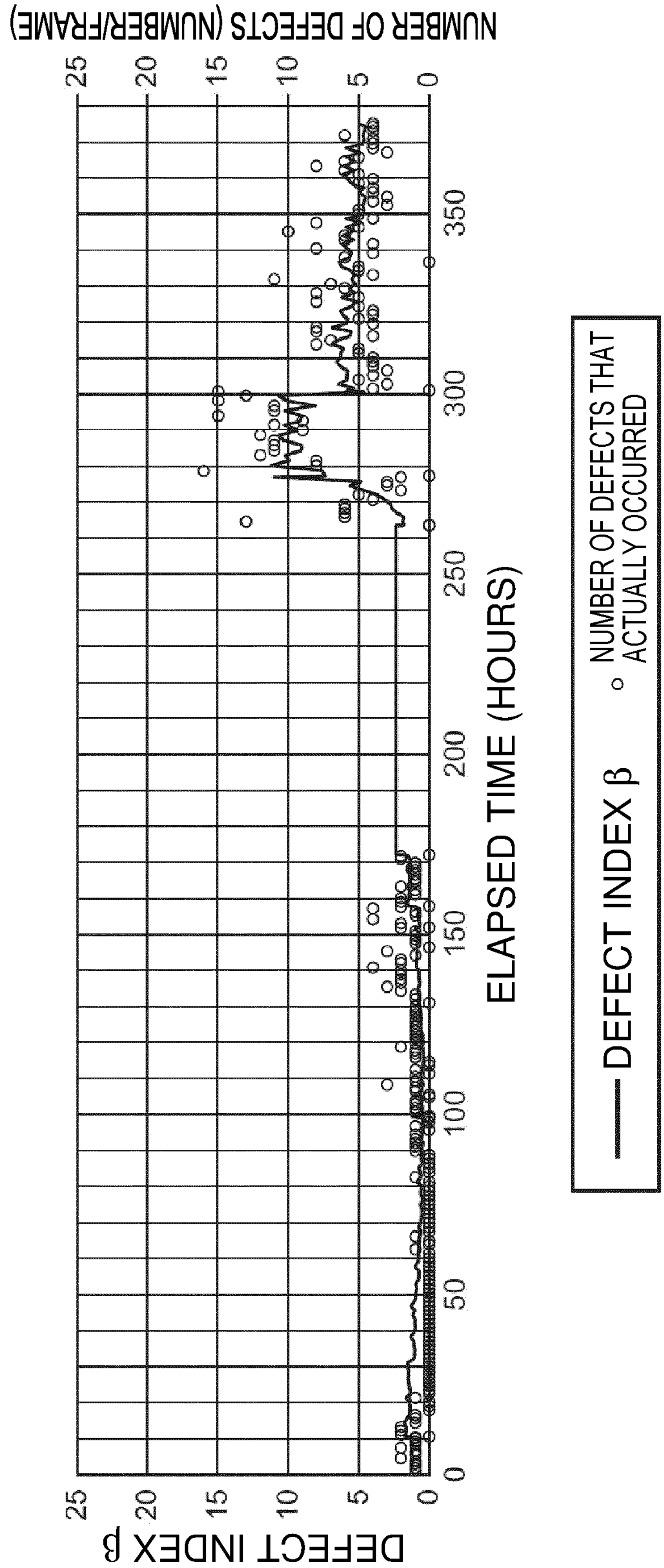
NUMBER OF DEFECTS (NUMBER/FRAME)
25
20
15
10
5
0
DEFECT INDEX β
0
50
100
150
200
250
300
350
ELAPSED TIME (HOURS)
DEFECT INDEX β
NUMBER OF DEFECTS THAT ACTUALLY OCCURRED

Fig. 18

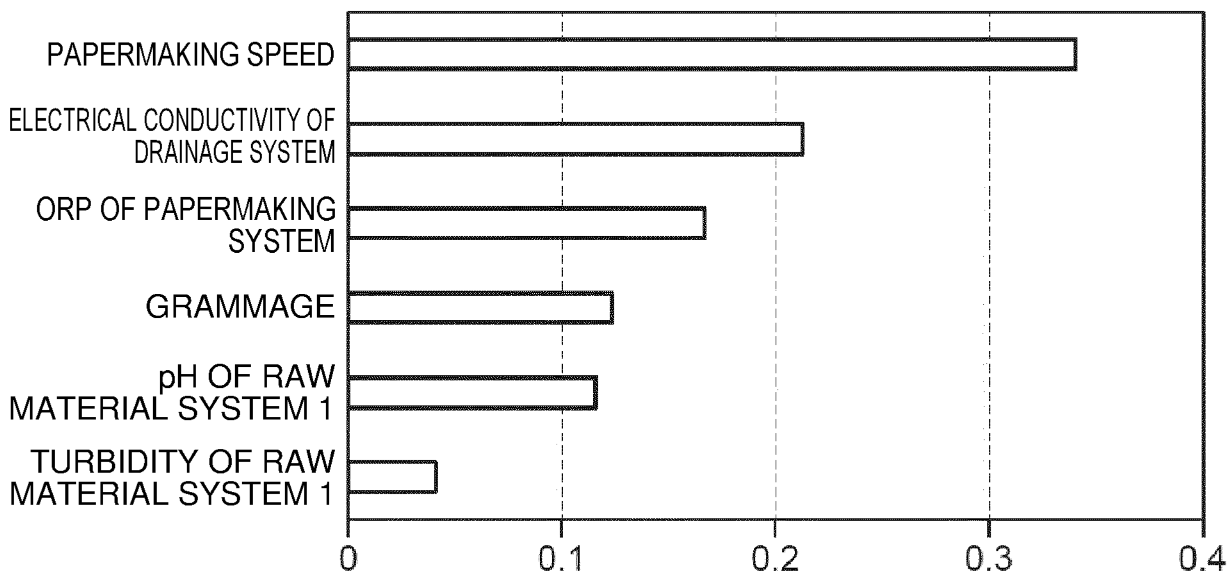
PAPERMAKING SPEED
ELECTRICAL CONDUCTIVITY OF DRAINAGE SYSTEM
ORP OF PAPERMAKING SYSTEM
GRAMMAGE
pH OF RAW MATERIAL SYSTEM 1
TURBIDITY OF RAW MATERIAL SYSTEM 1
0
0.1
0.2
0.3
0.4

Fig. 19

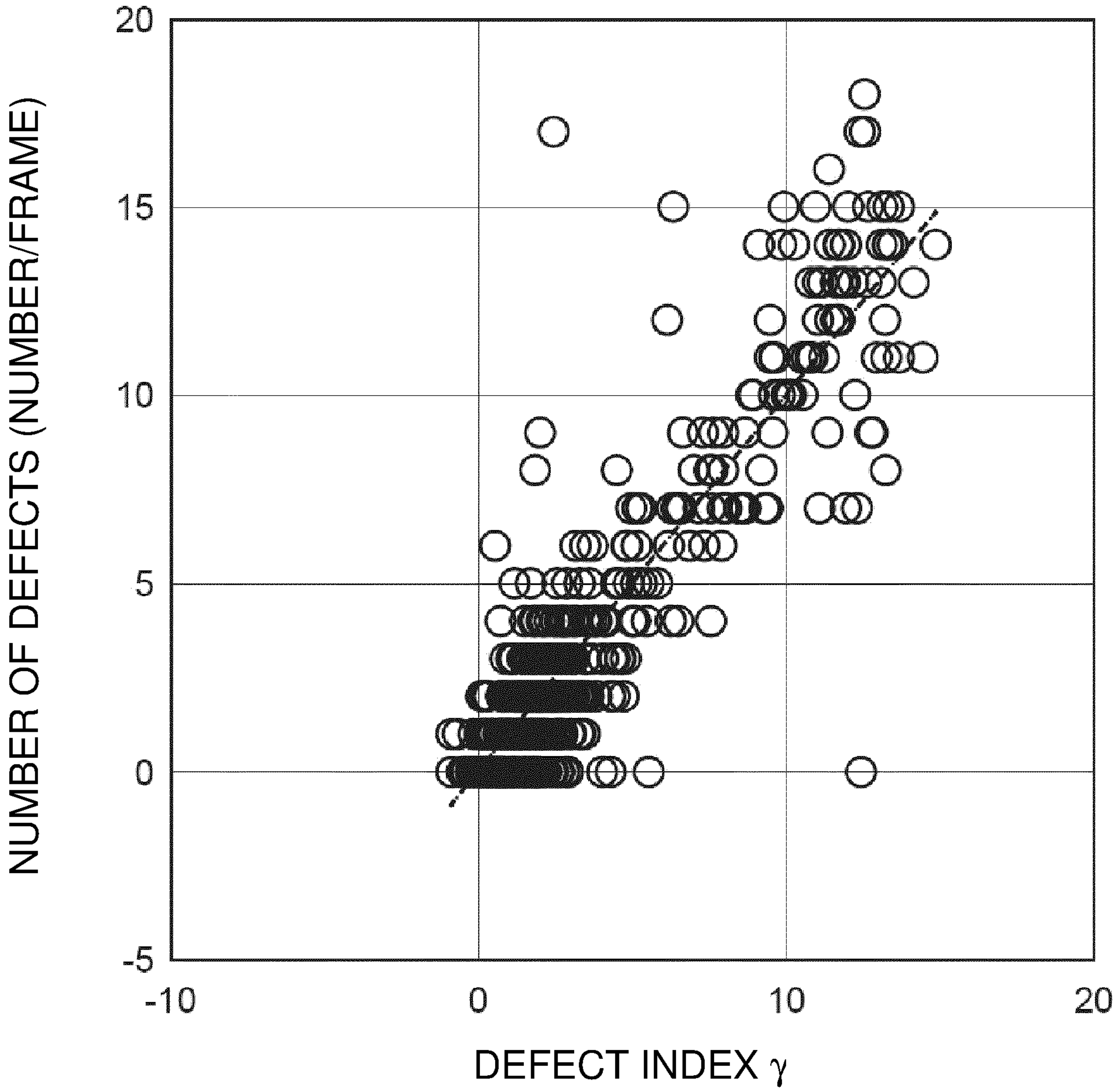
NUMBER OF DEFECTS (NUMBER/FRAME)
20
15
10
5
0
-5
-10
0
10
20
DEFECT INDEX γ

Fig. 20

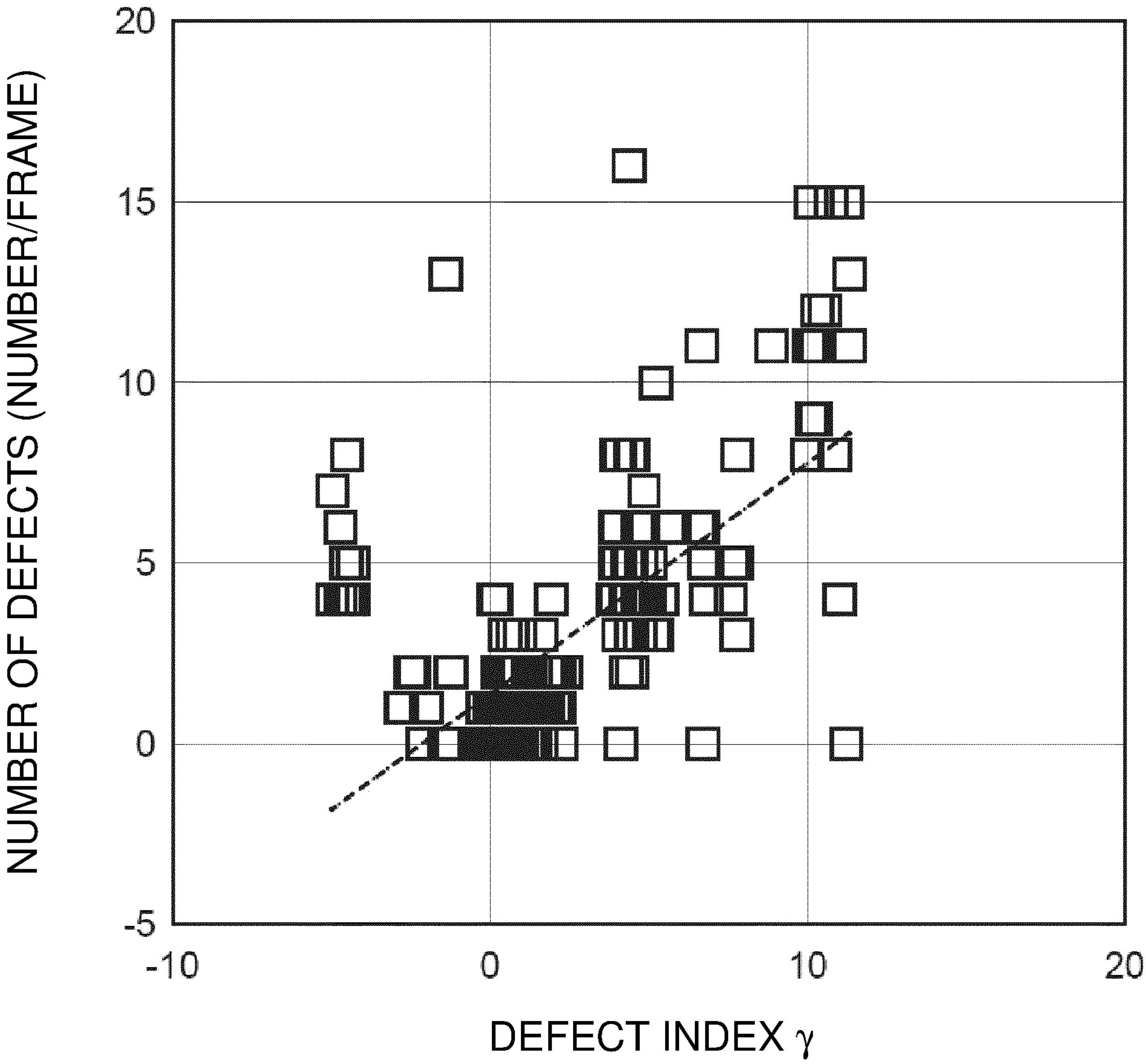
20
15
10
5
0
-5
-10
0
10
20
NUMBER OF DEFECTS (NUMBER/FRAME)
DEFECT INDEX γ

Fig. 21

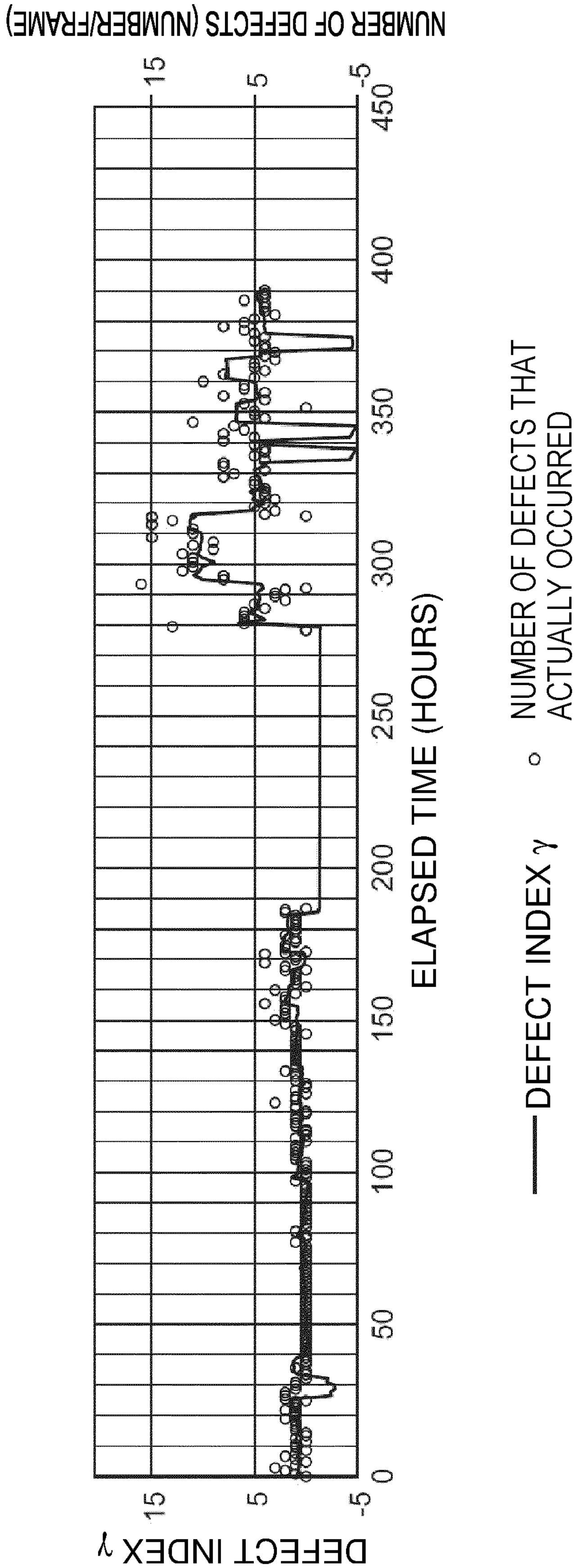

NUMBER OF DEFECTS (NUMBER/FRAME)
DEFECT INDEX γ
ELAPSED TIME (HOURS)
0
50
100
150
200
250
300
350
400
450
15
5
-5
DEFECT INDEX γ
NUMBER OF DEFECTS THAT ACTUALLY OCCURRED

Fig. 22

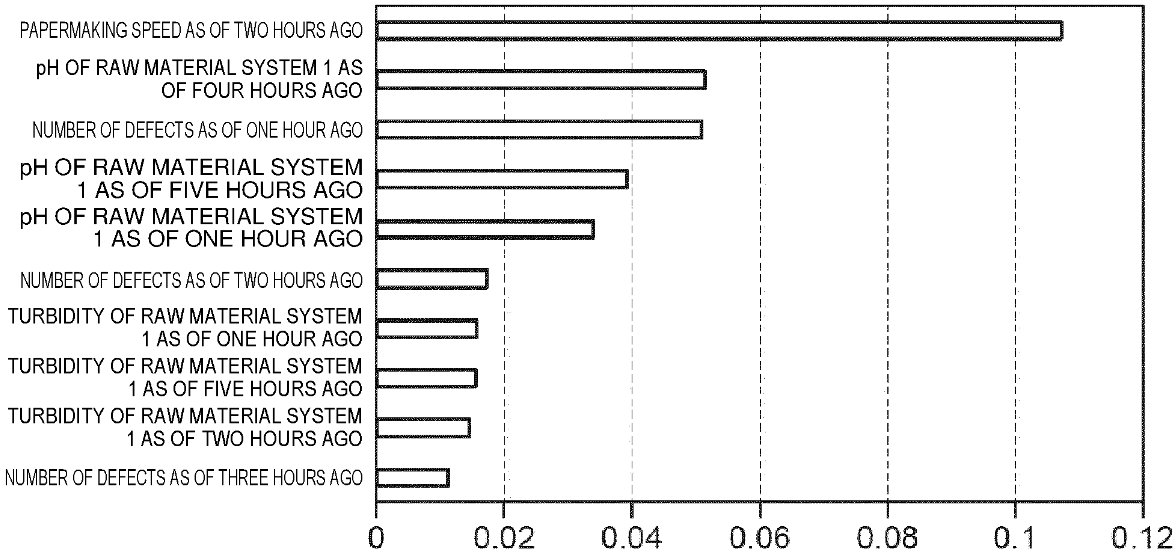
PAPERMAKING SPEED AS OF TWO HOURS AGO
pH OF RAW MATERIAL SYSTEM 1 AS OF FOUR HOURS AGO
NUMBER OF DEFECTS AS OF ONE HOUR AGO
pH OF RAW MATERIAL SYSTEM 1 AS OF FIVE HOURS AGO
pH OF RAW MATERIAL SYSTEM 1 AS OF ONE HOUR AGO
NUMBER OF DEFECTS AS OF TWO HOURS AGO
TURBIDITY OF RAW MATERIAL SYSTEM 1 AS OF ONE HOUR AGO
TURBIDITY OF RAW MATERIAL SYSTEM 1 AS OF FIVE HOURS AGO
TURBIDITY OF RAW MATERIAL SYSTEM 1 AS OF TWO HOURS AGO
NUMBER OF DEFECTS AS OF THREE HOURS AGO
0
0.02
0.04
0.06
0.08
0.1
0.12

Fig. 23

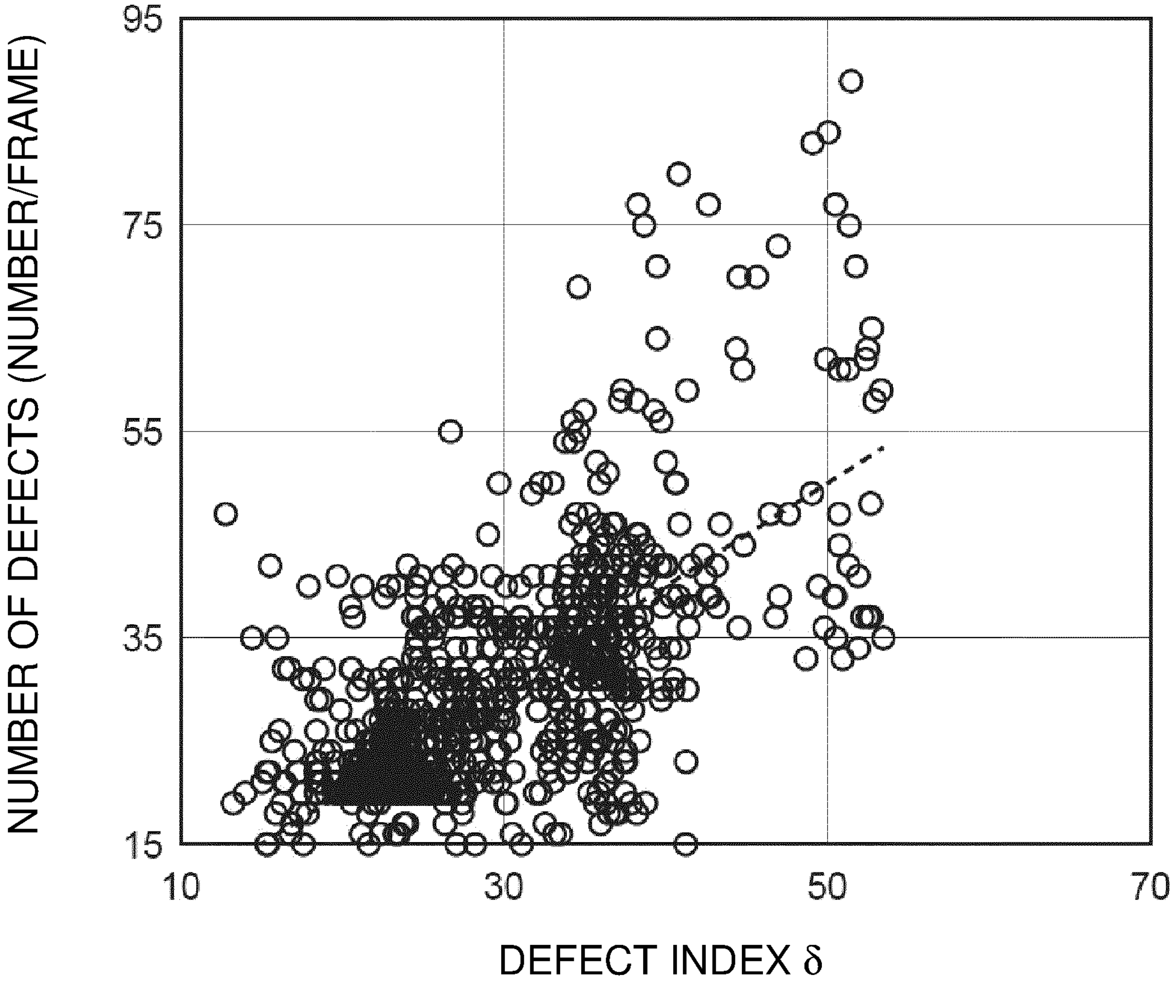
NUMBER OF DEFECTS (NUMBER/FRAME)
95
75
55
35
15
10
30
50
70
DEFECT INDEX δ

Fig. 24

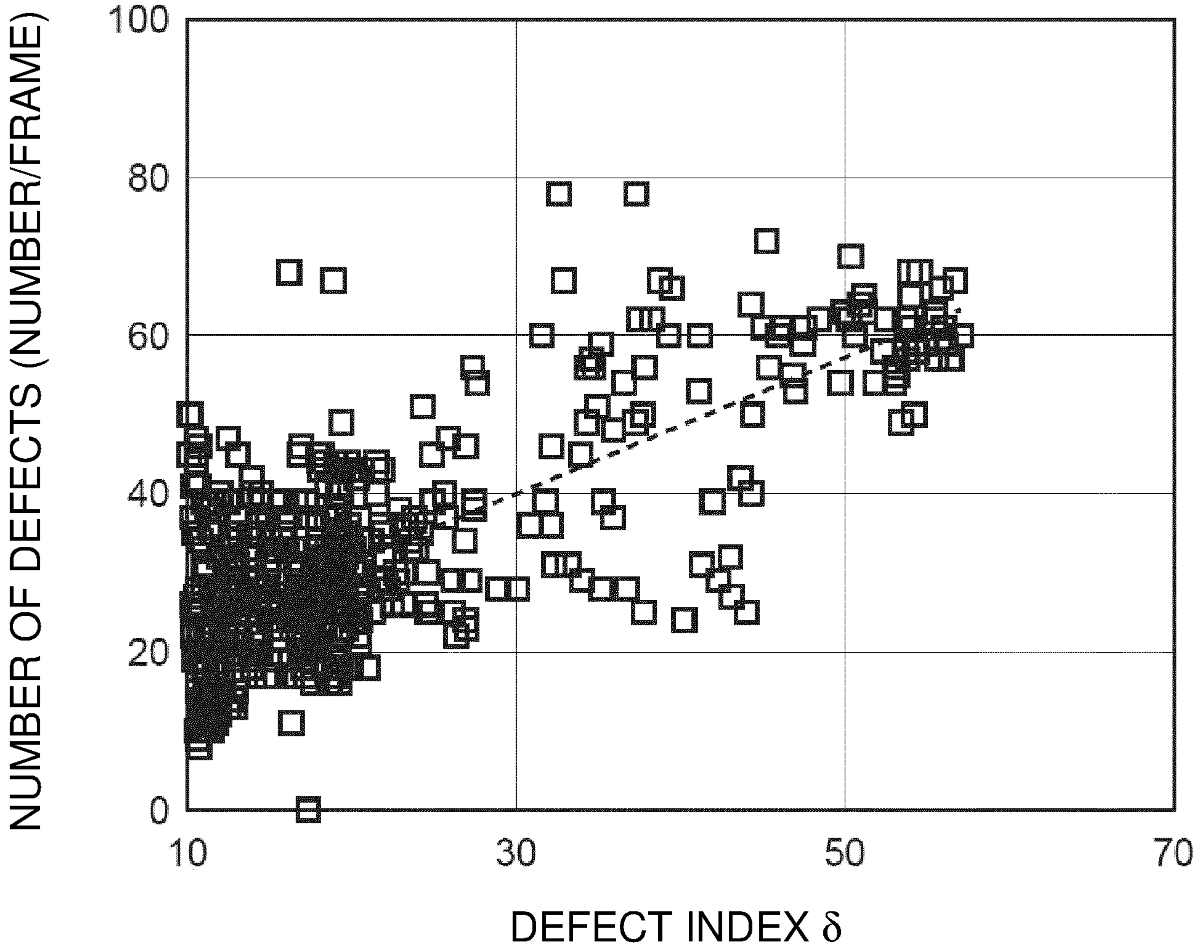
NUMBER OF DEFECTS (NUMBER/FRAME)
100
80
60
40
20
0
10
30
50
70
DEFECT INDEX δ

Fig. 25

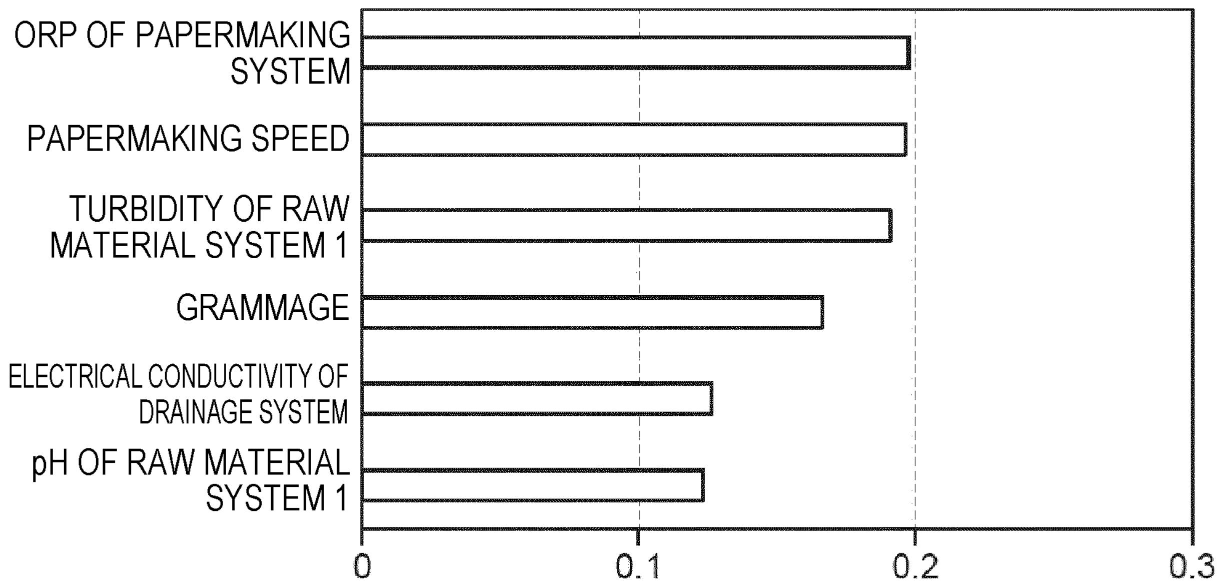
ORP OF PAPERMAKING SYSTEM
PAPERMAKING SPEED
TURBIDITY OF RAW MATERIAL SYSTEM 1
GRAMMAGE
ELECTRICAL CONDUCTIVITY OF DRAINAGE SYSTEM
pH OF RAW MATERIAL SYSTEM 1
0
0.1
0.2
0.3

Fig. 26

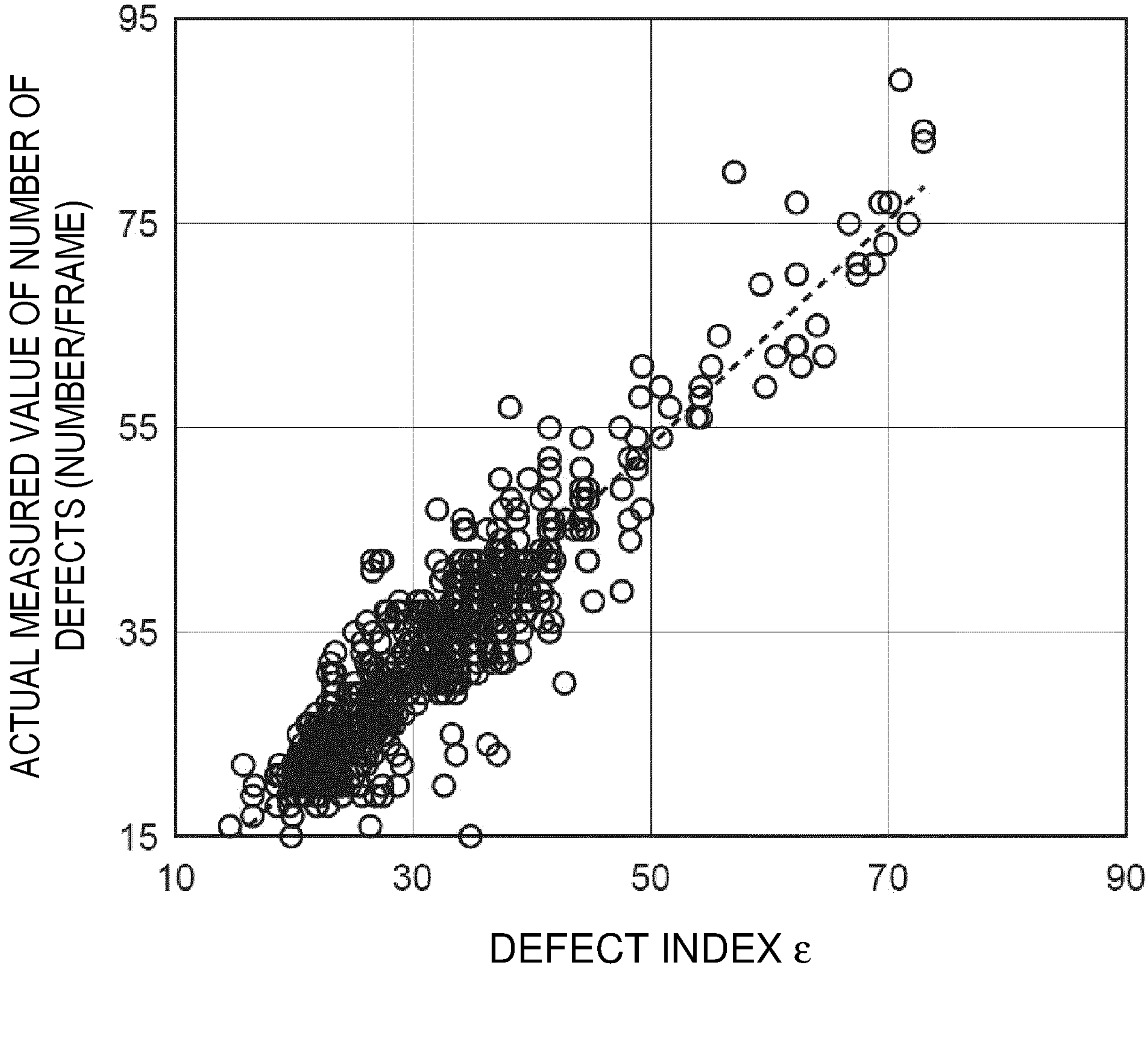
95
75
55
35
15
10
30
50
70
90
ACTUAL MEASURED VALUE OF NUMBER OF DEFECTS (NUMBER/FRAME)
DEFECT INDEX ε

Fig. 27

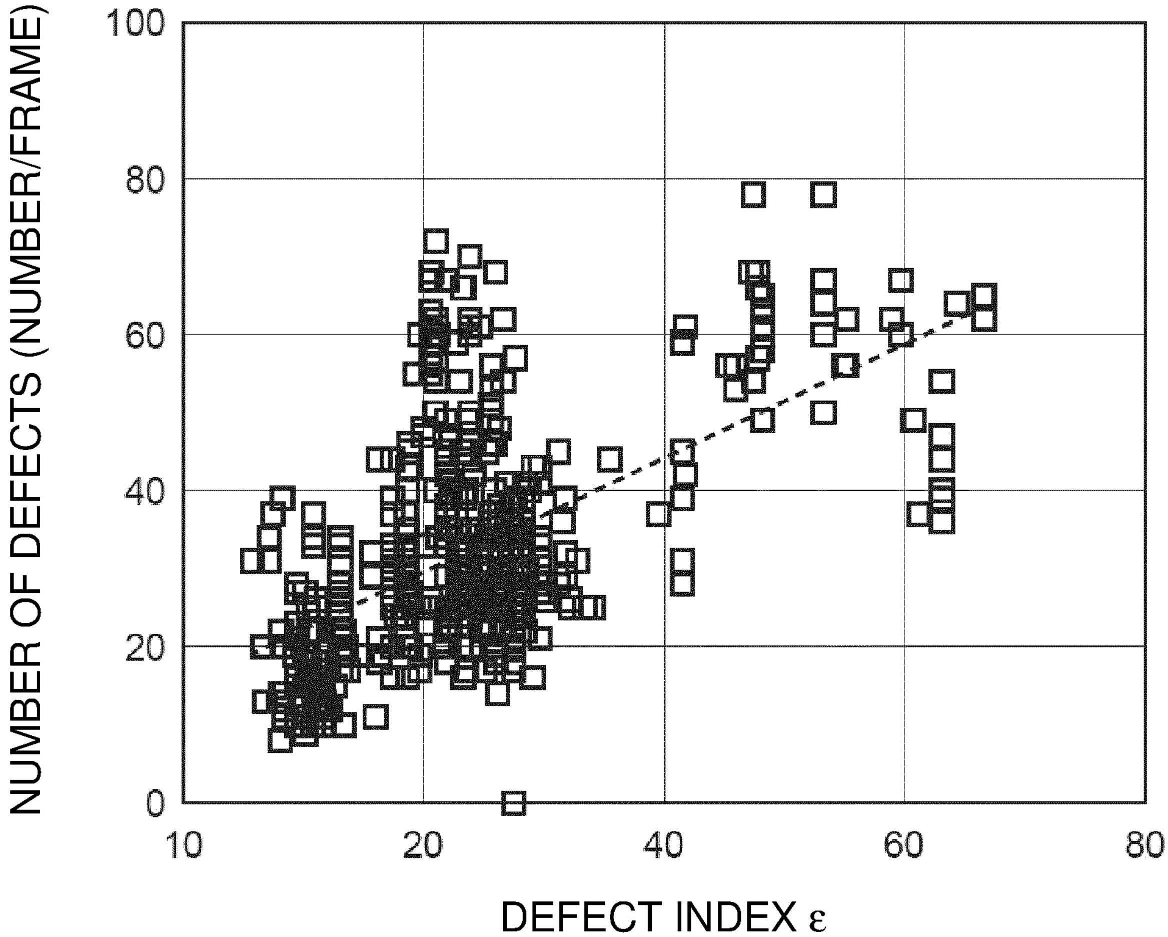
100
80
60
40
20
0
NUMBER OF DEFECTS (NUMBER/FRAME)
10
20
40
60
80
DEFECT INDEX ε

Fig. 28

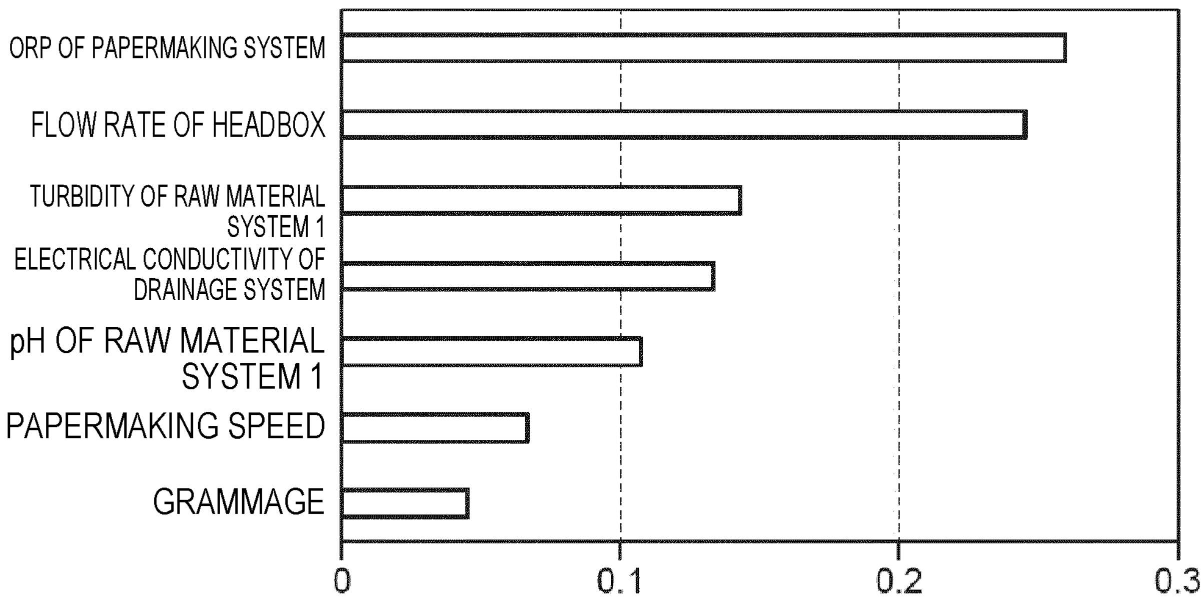
ORP OF PAPERMAKING SYSTEM
FLOW RATE OF HEADBOX
TURBIDITY OF RAW MATERIAL SYSTEM 1
ELECTRICAL CONDUCTIVITY OF DRAINAGE SYSTEM
pH OF RAW MATERIAL SYSTEM 1
PAPERMAKING SPEED
GRAMMAGE
0
0.1
0.2
0.3

| INTERNATIONAL SEARCH REPORT | International application No. PCT/JP2022/004611 |
|---|---|

**A. CLASSIFICATION OF SUBJECT MATTER**

***D21F 7/00***(2006.01)i; ***C02F 1/00***(2006.01)i
FI: D21F7/00 Z; C02F1/00 V

According to International Patent Classification (IPC) or to both national classification and IPC

**B. FIELDS SEARCHED**

Minimum documentation searched (classification system followed by classification symbols)

D21F7/00; C02F1/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 3-238346 A (NIPPON KAMIPARUPU KENKYUSHO KABUSHIKI KAISHA) 24 October 1991 (1991-10-24)<br>claims 2, 4, page 3, lower right column, lines 17-19 | 1-9 |
| X | JP 2019-193916 A (TOSHIBA CORPORATION) 07 November 2019 (2019-11-07)<br>claims 1-3, paragraphs [0002], [0009], [0043], [0044] | 1-9 |
| X | WO 03/074784 A1 (UNIVERSITY OF MANCHESTER INSTITUTE OF SCIENCE & TECHNOLOGY) 12 September 2003 (2003-09-12)<br>claims 9, 12, page 7, lines 11-23 | 1-9 |
| X | US 2003/0045962 A1 (ERYURER, Evren) 06 March 2003 (2003-03-06)<br>claims 1-5, paragraph [0002] | 1-9 |
| A | WO 2012/070644 A1 (KURITA WATER INDUSTRIES LIMITED) 31 May 2012 (2012-05-31) | 1-9 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 March 2022** | **29 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/004611**

| Patent document cited in search report | Publication date (day/month/year) | Patent family member(s) | Publication date (day/month/year) |
|---|---|---|---|
| JP 3-238346 A | 24 October 1991 | (Family: none) | |
| JP 2019-193916 A | 07 November 2019 | WO 2019/212004 A1 | |
| WO 03/074784 A1 | 12 September 2003 | GB 205336 D<br>AU 2003209464 A | |
| US 2003/0045962 A1 | 06 March 2003 | (Family: none) | |
| WO 2012/070644 A1 | 31 May 2012 | CN 103370471 A<br>KR 10-2013-0102608 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012070644 A